(19) Europäisches Patentamt
European Patent Office
Office européen des brevets

(11) **EP 3 501 561 B1**

(12) **EUROPEAN PATENT SPECIFICATION**

(45) Date of publication and mention
of the grant of the patent:
**23.12.2020 Bulletin 2020/52**

(51) Int Cl.:
*A61M 1/02* *(2006.01)*  *A61M 1/36* *(2006.01)*
*B01D 39/16* *(2006.01)*  *B32B 5/02* *(2006.01)*
*B32B 27/08* *(2006.01)*  *B32B 27/30* *(2006.01)*
*B32B 27/36* *(2006.01)*

(21) Application number: **17841436.3**

(22) Date of filing: **09.08.2017**

(86) International application number:
**PCT/JP2017/028929**

(87) International publication number:
**WO 2018/034213 (22.02.2018 Gazette 2018/08)**

(54) **FILTER ELEMENT FOR BLOOD TREATMENT FILTER, BLOOD TREATMENT FILTER, AND LEUKOCYTE REMOVAL METHOD**

FILTERELEMENT FÜR BLUTBEHANDLUNGSFILTER, BLUTBEHANDLUNGSFILTER UND VERFAHREN ZUR LEUKOZYTENENTFERNUNG

ÉLÉMENT DE FILTRE POUR FILTRE DE TRAITEMENT DU SANG, FILTRE DE TRAITEMENT DU SANG ET PROCÉDÉ D'ÉLIMINATION DE LEUCOCYTES.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.08.2016 JP 2016160732**

(43) Date of publication of application:
**26.06.2019 Bulletin 2019/26**

(73) Proprietor: **Asahi Kasei Medical Co., Ltd.**
**Tokyo 1000006 (JP)**

(72) Inventor: **SHIMADA, Nobukazu**
**Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A1- 0 554 460**    **EP-A1- 2 055 329**
**WO-A1-2007/145328**    **WO-A1-2015/088019**
**WO-A1-2016/013540**    **JP-A- H05 170 654**
**JP-A- H06 178 807**    **JP-A- H06 269 499**
**JP-A- 2006 280 483**    **JP-A- 2007 054 212**
**JP-B2- 4 108 393**    **US-A- 5 498 336**

**Description**

Technical Field

The present invention relates to a blood processing filter for removing unfavorable components such as aggregates and leukocytes from blood, i.e., blood component-containing liquids such as whole blood and blood products (liquids obtained by preparation from whole blood, and these liquids supplemented with various additives), a filter element therefor, and a leukocyte removal method using the blood processing filter.

Background Art

In the field of blood transfusion, so-called blood component transfusion of separating a blood component necessary for a recipient from a whole blood product and transfusing the blood component has generally been practiced in addition to so-called whole blood transfusion of transfusing a whole blood product in which blood collected from a donor is supplemented with an anticoagulant. The blood component transfusion includes red cell transfusion, platelet transfusion, plasma transfusion, and the like depending on the type of the blood component necessary for a recipient, and the blood product used for these transfusions includes a red cell product, a platelet product, a plasma product, and the like.

Furthermore, so-called leukocyte-free blood transfusion of transfusing a blood product after removing leukocytes contained in the blood product has become widespread recently. This is because it has been revealed that relatively slight adverse reactions accompanying blood transfusion, such as headache, nausea, chill, or febrile non-hemolytic reaction, and severe adverse reactions having serious effects on a recipient, such as alloantigen sensitization, viral infection, or post-transfusion GVHD, are mainly caused by leukocytes contained in the blood product used in blood transfusion. For preventing relatively slight adverse reactions such as headache, nausea, chill, or fever, it is considered necessary to remove leukocytes in the blood product until the residual rate becomes from $10^{-1}$ to $10^{-2}$ or less. Also, for preventing alloantigen sensitization or viral infection, which is a severe adverse reaction, it is considered necessary to remove leukocytes until the residual rate becomes from $10^{-4}$ to $10^{-6}$ or less.

Furthermore, in recent years, leukocyte removal therapy by the extracorporeal circulation of blood has been practiced in the treatment of diseases such as rheumatism or ulcerative colitis, and high clinical effects have been obtained.

Currently, methods of removing leukocytes from the blood product are roughly classified into two types: a centrifugation method of separating and removing leukocytes by using a centrifuge and utilizing the difference in specific gravity among blood components, and a filter method of removing leukocytes by using a filter element consisting of a fiber assembly such as a nonwoven fabric or a porous structure having continuous pores, or the like. The filter method which removes leukocytes by adhesion or adsorption is most widely used at present because of having the advantages that the operation is simple and the cost is low, for example.

In recent years, new demands for leukocyte removal filters have been proposed in the medical practice. One of the demands is to prevent anaphylactoid adverse reactions of blood transfusion when a blood product filtered through the filter is transfused to a patient.

In general, a material made of polyester may be used as a filter element because of its inexpensiveness and easy production. This material contains carboxylic acid in a molecular chain and is therefore negatively charged in a neutral to weakly alkaline region such as blood. In this context, upon contact with a filter element having negatively charged surface, blood containing plasma proteins may produce a large amount of bradykinin. Since bradykinin is a substance having a blood pressure lowering effect, its production in a large amount is not preferred. Bradykinin is produced, probably, because the contact of a negatively charged material with blood activates blood coagulation factor XII, and the activated blood coagulation factor XII forms, from prekallikrein, kallikrein, which further causes the limited degradation of high-molecular-weight kininogen to form (produce) bradykinin.

As for such a problem, for example, Patent Literature 1 discloses a technique of reducing bradykinin production upon blood contact by modifying the surface of a filter element made of polyolefin by radiation or the like.

Patent Literature 2 discloses that a blood processing filter having a filter element with a low amount of bradykinin produced in blood after filtration can be prepared by accurately quantifying the negative charge of the surface of the filter element itself (except for a surface coating material), thereby controlling the negative charge of the filter element itself to a predetermined level or lower, and further positively charging the surface using a positively charged coating material.

WO2015/088019 provides a protocol for the preparation of a non-woven fabric consisting of polybutylene terephthalate fiber and having a mass per unit area of 22 g/m$^2$, a thickness of 0.13 mm, a filling rate of 0.12 and an average fiber diameter of 1.0 μm. The document also discloses further subjecting the nonwoven fabric to coating with a hydrophilic polymer and using the nonwoven fabric having the coat layer formed by coating as a leukocyte removal filter material.

Citation List

Patent Literature

Patent Literature 1: Japanese Patent No. 4261623
Patent Literature 2: Japanese Patent Laid-Open No. 2007-054212

Summary of Invention

Technical Problem

However, even if a filter element is modified at its surface according to the description of Patent Literature 1, bradykinin production is not sufficiently reduced in actuality. Thus, such a filter element is still practically unsuitable.
On the other hand, mere conformity with the description of Patent Literature 2 results in low leukocyte removal performance, though bradykinin production can be suppressed. Thus, such a filter or a filter element has been found to be practically unsuitable.
In light of the problems of the conventional techniques, an object of the present invention is to provide a blood processing filter that reduces the production of bradykinin and the like during blood filtration even while achieving favorable filtration performance (i.e., high removal performance for leukocytes and the like and a short filtration time).

Solution to Problem

The present inventor has conducted diligent studies and consequently found that as the production of bradykinin is closely related to the surface charge of a nonwoven fabric contained in a filter element, the control of the surface charge of the nonwoven fabric also influences the filtration performance itself of the nonwoven fabric. The present inventor has further found that when the carboxyl group equivalent of a nonwoven fabric contained in a filter element falls within a specific range, the surface charge of the nonwoven fabric can be controlled to positive charge without largely influencing filtration performance, and this can drastically reduce bradykinin production and also maintain high removal performance for leukocytes and the like as compared with conventional filter elements.
Specifically, the present invention is as follows:

[1] A filter element for a blood processing filter, comprising a nonwoven fabric, wherein the nonwoven fabric has a carboxyl group equivalent of from 20 to 140 $\mu$eq/g and a surface $\zeta$ potential of 0 mV or larger.
[2] The filter element for a blood processing filter according to [1], wherein the carboxyl group equivalent of the nonwoven fabric is from 54 to 140 $\mu$eq/g .
[3] The filter element for a blood processing filter according to [1] or [2], wherein the filter element comprises the nonwoven fabric having a basic nitrogen-containing functional group in a surface portion.
[4] The element for a blood processing filter according to [3], wherein the surface portion further has a nonionic group, and a ratio of an amount of substance of the basic nitrogen-containing functional group to a total amount of substance of the nonionic group and the basic nitrogen-containing functional group is from 0.2 to 50.0% by mol.
[5] The filter element for a blood processing filter according to any of [1] to [4], wherein the nonwoven fabric comprises a fiber material, and the carboxyl group equivalent of the fiber material is from 20 to 140 $\mu$eq/g .
[6] The filter element for a blood processing filter according to any of [1] to [5], wherein the fibers of the nonwoven fabric are made of a polyester resin.
[7] The filter element for a blood processing filter according to any of [1] to [6], wherein the nonwoven fabric comprises a coat layer.
[8] A blood processing filter comprising a filter element according to any of [1] to [7].
[9] A leukocyte removal method comprising a step of allowing a leukocyte-containing liquid to pass through a blood processing filter according to [8], under the proviso that methods for the treatment of the human and animal body by surgery and therapy are excluded.

Advantageous Effects of Invention

Use of the filter element of the present invention can provide a blood processing filter that drastically reduces bradykinin production during blood filtration and maintains, for example, high removal performance for leukocytes and the like, as compared with conventional filters.

Brief Description of Drawings

[Figure 1] Figure 1 is a schematic view of a blood processing filter equipped with a filter element for a blood processing filter according to one embodiment of the present invention.
[Figure 2] Figure 2 is a cross-sectional view of a blood processing filter equipped with a filter element for a blood processing filter according to one embodiment of the present invention.

Description of Embodiments

Hereinafter, a mode for carrying out the present invention (hereinafter, referred to as "the present embodiment") will be described in detail.

In the present embodiment, the filter element comprises a nonwoven fabric. The filter element may comprise one nonwoven fabric or may comprise a plurality of nonwoven fabrics. Alternatively, the filter element may comprise the nonwoven fabric in combination with an additional sheet.

When the filter element comprises a plurality of nonwoven fabrics, the plurality of nonwoven fabrics may be of single type or may be of plural types. At least one of the nonwoven fabrics can satisfy the conditions involving a carboxyl group equivalent of from 20 to 140 ($\mu$eq/g) and a surface $\zeta$ potential of 0 mV or larger. It is preferred that all the nonwoven fabrics should satisfy the conditions.

In the present embodiment, the nonwoven fabric may only consist of a fiber material or may have a coat layer or the like in the surface portion of the fiber material.

In the present embodiment, the material for the nonwoven fabric (fiber material) is not particularly limited and may be, for example, resin fibers formed by spinning a polyester resin such as polyethylene terephthalate (PET) or polybutylene terephthalate (PBT).

The blood processing filter of the present embodiment comprises, for example, a filter element and a container for housing the filter element.

For example, the blood processing filter can be configured to comprise a filter element and an inlet-side container material and an outlet-side container material disposed to sandwich the filter element, wherein the inlet-side container material and the outlet-side container material have holding parts for holding the filter element by grasping its outer edges.

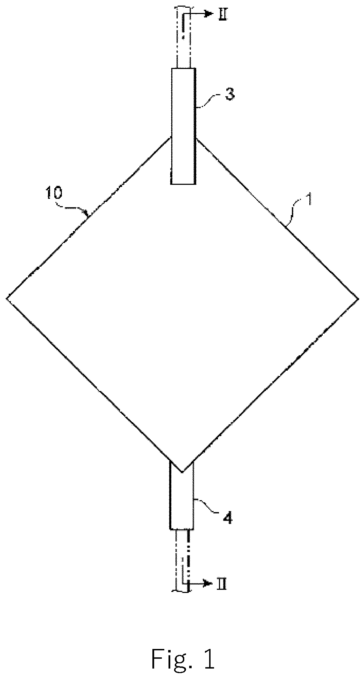

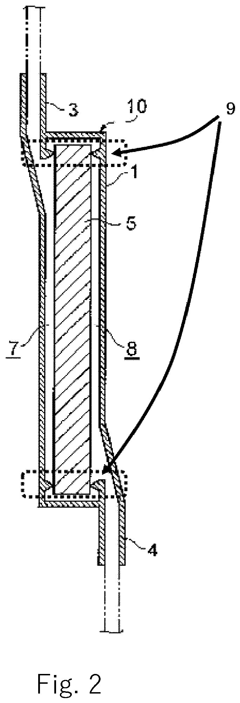

Figure 1 is a schematic view of such a blood processing filter (leukocyte removal filter), and Figure 2 is a cross-sectional view taken along the II-II line of Figure 1.

As shown in Figures 1 and 2, a blood processing filter 10 has a flat container 1 and a blood processing filter element 5 in a substantially dry state housed in the inside thereof. The container 1 which houses the blood processing filter element 5 comprises two elements: an inlet-side container material having a first port 3 at the end part of one principal surface; and an outlet-side container material having a second port 4 at the end part of the other principal surface. The space within the flat container 1 is partitioned by the blood processing filter element 5 into space 7 on the first port side and space 8 on the second port side.

This blood processing filter 1 has a structure where the inlet-side container material and the outlet-side container material are disposed to sandwich the filter element 5, and these two container materials hold the filter element 5 such that their respective holding parts disposed on a portion of them grasp outer edges 9 of the filter element 5.

Also, in the present embodiment, the blood processing filter may have a structure where the filter element is joined with the container by welding or the like so that the filter element is held by the container.

In the present embodiment, the nonwoven fabric contained in the filter element has a carboxyl group equivalent of from 20 to 140 ($\mu$eq/g) and a surface $\zeta$ potential of 0 mV or larger.

The filtration functions of the blood processing filter as a filter, such as aggregate removal performance (function of securing flowability while capturing aggregates in blood), removal performance for leukocytes and the like, and recovery performance for useful components (red cells, plasma proteins, etc.) are achieved by suitably controlling the physical properties and chemical properties of the filter element contained therein. In general, a nonwoven fabric is used in a portion of the filter element because of its high surface area to exert the functions described above.

During blood processing, the contact of plasma protein components in blood with a nonwoven fabric in a filter element activates a coagulation factor and consequently produces bradykinin. The post-filtration blood, when administered to a patient, may cause adverse reactions of blood transfusion, such as a drop in the blood of the patient. It is known that this bradykinin production is influenced by the negative charge of the nonwoven fabric surface.

In general, a polyester material such as PBT or PET is used, because of its inexpensiveness and easy availability, as a fiber material for a nonwoven fabric contained in a filter element. This polyester material contains a carboxyl group in a molecular chain and is therefore usually negatively charged in a state after spinning. Thus, for decreasing the absolute value of negative charge of the polyester nonwoven fabric, it is effective to suppress degradation by reducing the quantity of heat or pressure applied to a polyester resin during spinning and thereby reduce a carboxyl group equivalent exposed in the course of degradation to attain the small absolute value of negative charge.

In order to decrease the absolute value of negative charge, it is also possible to prepare a coated nonwoven fabric by coating a fiber material with a positively charged coating material.

However, the studies of the present inventor have revealed that when the carboxyl group equivalent of the nonwoven fabric thus coated with a coating material is larger than a predetermined value (140 $\mu$eq/g), the amount of coating on the fiber material coated with the coating material for positively charging the nonwoven fabric is large, and the surface coating is non-uniform, thereby deteriorating filtration performance such as leukocyte removal performance or aggregate removal performance.

Too high a positive charge value of the nonwoven fabric activates a complement after contact with blood and has the risk of easily causing hemolysis of red cells. The studies of the present inventor have also revealed that when the carboxyl group equivalent of the nonwoven fabric thus coated with a coating material is less than a predetermined value (less than 20 $\mu$eq/g), the positive charge value of the whole nonwoven fabric is too high and causes such a risk of hemolysis.

On the basis of the findings described above, the present inventor has found that: use of a nonwoven fabric having a carboxyl group equivalent of from 20 to 140 ($\mu$eq/g) and a surface $\zeta$ potential of 0 mV or larger as a nonwoven fabric contained in a filter element can prevent reduction in filtration performance caused by the thickness of the average fiber diameter of a fiber material, and optimize the balance between the amount of coating and the total charge of the coated nonwoven fabric even when a positively charged coating material is used in the coating; and as a result, a blood processing filter that can achieve both of filtration performance and the improved quality of the resulting blood product can be provided.

In this context, the carboxyl group equivalent is the number of carboxyl groups contained per g of the nonwoven fabric. When the nonwoven fabric has a coat layer, the carboxyl group equivalent is a value based on the sum of carboxyl groups contained in the fibers constituting the fiber material and carboxyl groups contained in the coating material constituting the coat layer. The coating material is positively charged, but may contain a carboxyl group in rare cases. In this case, the coat layer is richer in a positively charged functional group such as an amino group, for example, and is thereby positively charged as a whole, though the coating material is partially negatively charged. In the case of using such a coating material containing a carboxyl group, the amount of coating with the coating material required to positively charge the surface of the nonwoven fabric tends to be large, as compared with the case of using a coating material containing no carboxyl group. For both the coating material containing a carboxyl group and the coating material containing no carboxyl group, a carboxyl group equivalent of less than 20 ($\mu$eq/g) has the risk of hemolysis as a result of elevating the positive charge value of the whole nonwoven fabric and reduces filtration performance due to the thick average fiber diameter of the fiber material, as mentioned above. In this respect, when the carboxyl group equivalent of the whole nonwoven fabric also including the coat layer is from 20 to 140 ($\mu$eq/g), the situation described above can be taken into consideration, and both of filtration performance and improved quality of the resulting blood product can be achieved for both the coating material containing a carboxyl group and the coating material containing no carboxyl group.

Understandably, when the mass of the coating material is 0.1% by mass or less relative to the mass of the fibers constituting the fiber material of the nonwoven fabric or when the coating material contains no carboxyl group, the carboxyl group equivalent of the nonwoven fabric is determined by the carboxyl group equivalent of the fiber material. In this case, it is effective to control the carboxyl group equivalent of the nonwoven fabric by adjusting spinning conditions for the fiber material, as mentioned above.

In the present embodiment, it is desirable that the carboxyl group equivalent of the nonwoven fabric contained in the filter element should be preferably from 30 to 140 $\mu$eq/g, more preferably from 40 to 140 $\mu$eq/g, further preferably from 54 to 140 $\mu$eq/g, further preferably from 54 to 120 $\mu$eq/g, further preferably from 60 to 120 $\mu$eq/g, further preferably from 80 to 120 $\mu$eq/g, most preferably from 90 to 110 $\mu$eq/g.

In this context, it is more desirable for the filter element to set the carboxyl group equivalent to a higher region within the range from 20 to 140 $\mu$eq/g, for the following two reasons:

1) In the case of producing the fiber material of the nonwoven fabric by melt spinning according to a melt blown method, it is necessary for elevating the carboxyl group equivalent of the nonwoven fabric to stretch a resin as the fiber material at a higher quantity of heat or pressure in order to cause the degradation of the resin during spinning to some extent. This can render an average fiber diameter finer and enhances leukocyte removal performance.
2) Even use of the coating material in a larger amount does not render a $\zeta$ potential too high. Therefore, the elevated coating rate of the coat layer can achieve sufficient hydrophilization. This can prevent drift upon contact with blood and enhance filtration performance.

As mentioned above, in the present embodiment, the carboxyl group equivalent of the nonwoven fabric is the number of carboxyl groups contained per g of the nonwoven fabric. For example, when the nonwoven fabric is made of only polyester, the carboxyl group equivalent of the nonwoven fabric is $n \times M$ wherein n represents the number of carboxyl groups contained in one molecule of the polyester constituting the nonwoven fabric, and M represents the molar number of the polyester contained in g of the fibers.

The carboxyl group equivalent of the nonwoven fabric can be measured according to the following procedures 1) to 3):

1) Approximately 0.3 g of the nonwoven fabric pre-dried (80°C, overnight), and 5 mL of benzyl alcohol are added to a glass container and heated for from 5 to 15 minutes in an oil bath of 195°C to completely dissolve the nonwoven fabric (coat layer and fiber material). To the solution, 80 mL of chloroform is added to prepare a sample solution.

2) The sample solution obtained in the preceding procedure 1) is titrated with a 0.01 mol/L solution of sodium hydroxide in benzyl alcohol using a potentiometric titration apparatus (e.g., AT-500N (manufactured by Kyoto Electronics Manufacturing Co., Ltd.); the electrode used is a composite glass electrode). The titration is terminated upon reaching the inflection point. In this context, when the inflection point cannot be clearly read in the potentiometric titration, bromothymol blue-phenol red is used as an indicator at the same time with the potentiometric titration to confirm that the color is changed from yellow to purple.

3) The test is conducted in the same way as in the procedure 2) using a blank sample solution prepared in the same way as above except that the nonwoven fabric is not dissolved therein. The carboxyl group equivalent is calculated according to the following expression:

```
<Expression>

Carboxyl group equivalent (μeq/g)

= (V₁ - V₀) × 0.01 × f / (S × 1000) × 10⁶
```

In this context, each parameter is as follows:

S: mass (g) of a collected sample
$V_1$: amount (mL) of the 0.01 mol/L solution of sodium hydroxide in benzyl alcohol required for the present sample solution
$V_0$: amount (mL) of the 0.01 mol/L solution of sodium hydroxide in benzyl alcohol required for the blank sample solution
f: factor of the 0.01 mol/L solution of sodium hydroxide in benzyl alcohol

When the nonwoven fabric has a coat layer, it is preferred to control the carboxyl group equivalent of the fiber material contacted with the coating material to a predetermined value or higher, from the viewpoint of another effect.

Specifically, for example, in the case of using, as a coating material for the nonwoven fabric, for example, a polymer having a monomer unit having an ester structure such as 2-hydroxyethyl (meth)acrylate and a monomer unit having a basic nitrogen-containing functional group, the basic nitrogen-containing functional group works as a catalyst by the application of thermal energy of drying treatment or the like after coating treatment so that transesterification reaction occurs between the carboxyl group of the fiber material and the ester moiety such as 2-hydroxyethyl (meth)acrylate to cause cross-linking reaction. As a result, the coating material is stably supported on the fiber material while maintaining its chemical properties. Therefore, the risk of eluting the coating material during blood filtration is also reduced. Thus, the effect of improving blood quality is obtained.

In the present embodiment, the surface $\zeta$ potential of the nonwoven fabric having a carboxyl group equivalent of from 20 to 140 ($\mu$eq/g) is set to 0 mV or larger.

The surface $\zeta$ potential of the nonwoven fabric is an index that indicates the total surface charge of the nonwoven fabric. When the nonwoven fabric has a surface layer such as a coat layer, the surface $\zeta$ potential means the potential of the surface layer. If the surface potential of the nonwoven fabric differs between the front and back sides, the surface $\zeta$ potential is set to 0 mV or larger on both the front and back sides.

It is desirable that the surface $\zeta$ potential of the nonwoven fabric should be preferably larger than 0 mV, more preferably larger than 0 mV and 150 mV or smaller, further preferably larger than 0 mV and 100 mV or smaller, further preferably larger than 0 and 80 mV or smaller, further preferably 10 or larger and 70 mV or smaller, most preferably 15 or larger and 60 mV or smaller.

When the $\zeta$ potential is less than 0 mV, bradykinin is more likely to be produced during blood processing. Understandably, too high a $\zeta$ potential of the nonwoven fabric is not preferred from the viewpoint of blood quality because such too high a ζ potential of the nonwoven fabric in turn activates a complement such as C3a upon contact with blood and easily causes hemolysis of red cells.

The surface ζ potential of the nonwoven fabric can be adjusted to within the range described above, for example, by adjusting the carboxyl group equivalent of the nonwoven fabric and positive charge contained in the coat layer used for coating the nonwoven fabric.

In this context, the positive charge contained in the coat layer is determined by the mass of the coat layer and positive charge contained per unit mass of the coating material constituting the coat layer. In this context, the positive charge contained per unit mass of the coating material is higher when the amount of substance (mol/g) of a positive functional group contained in the coating material of unit mass is higher and charge (positive ion strength) contained in each positive functional group is higher.

For example, a polyethylene terephthalate nonwoven fabric (fiber material + coat layer) may have a carboxyl group equivalent of 30 μeq/g and have a coating material containing diethylaminoethyl methacrylate as a positive functional group. In this case, when the mass of the coat layer per g of the nonwoven fabric is 10.0 mg/g and the amount of substance of diethylaminoethyl methacrylate per unit mass of the coating material in the surface portion (surface portion of the coat layer) of the nonwoven fabric is $2.3 \times 10^{-4}$ mol/g, the actually measured value of the surface ζ potential of the nonwoven fabric is 40 mV. Thus, the ζ potential can be further elevated by elevating the amount of substance of diethylaminoethyl methacrylate contained in the coating material of unit mass or the mass of the coat layer per g of the nonwoven fabric.

For more reliably controlling the surface ζ potential of the nonwoven fabric, it is desirable to adjust the positive charge contained in the coat layer used for coating the nonwoven fabric, from the viewpoint of production cost or from the viewpoint of homogeneous coat layer formation.

In the present embodiment, the surface ζ potential of the nonwoven fabric can be measured by the following procedures:

1) The nonwoven fabric is cut into a size of approximately 1 cm × 2 cm and dipped in a 1 mM potassium chloride solution.

2) The nonwoven fabric thus dipped is loaded in a cell for plate samples, followed by electrophoresis using a ζ potential apparatus (e.g., ELS-Z Photal (manufactured by Otsuka Electronics Co., Ltd.)). In this context, the migration solution used is a dispersion of polystyrene latex particles in a 1 mM potassium chloride solution.

3) The sample thus electrophoresed is analyzed using software attached to the apparatus from the mobility of the latex particles shown in the spectrum to calculate the surface ζ potential (mV) of the nonwoven fabric.

The blood processing filter is usually subjected to sterilization treatment by a steam heat treatment method before use. In this respect, the physical structure of the nonwoven fabric contained in the filter element is thought to be largely changed by the steam heat treatment. Particularly, if the nonwoven fabric contracts in the planar direction, the holding parts in the blood processing filter having, for example, the structure as shown in Figure 1 mentioned above become structurally unstable to thereby reduce the removal performance for leukocytes and the like of the blood processing filter and handleability.

From this viewpoint, in the present embodiment, the quantity of heat of the uncrystallized form of the nonwoven fabric contained in the filter element is preferably set to 5 J/g or smaller before steam heat treatment. The "quantity of heat of the uncrystallized form" is an index that indicates the crystallinity of a resin. A smaller value of this "quantity of heat of the uncrystallized form" means higher crystallinity of the resin. The quantity of heat of the uncrystallized form is preferably 3 J/g or smaller, more preferably 2 J/g or smaller, further preferably 1 J/g or smaller.

This can suppress change in the physical properties of the nonwoven fabric in association with steam heat treatment or the like and maintain high removal performance for leukocytes and the like. In general, conditions for the steam heat treatment differ variously depending on kits incorporating the blood processing filter produced by each bag manufacture. The filter element of the present embodiment has thermally stable nature and therefore has heat stability that allows the blood processing filter to withstand a wider range of steam heat treatment conditions as compared with blood processing filters using conventional filter elements.

Use of the filter element comprising such a nonwoven fabric is also effective for improving filtration performance and handleability as a blood processing filter.

For example, in the filter in which the filter element is sandwiched and held by the rigid container as shown in Figures 1 and 2, the rebound strength of the filter element against the holding parts of the container is high even after steam heat treatment so that the strong holding state of the filter element by the container holding parts is maintained. This can suppress a phenomenon in which blood leaks through the gaps between the holding parts and the filter element and runs into the outlet space from the inlet space without passing through the filter element (side leak phenomenon). Thus, the effect of improving removal performance for leukocytes and the like is obtained.

The value obtained by subtracting the quantity of heat of the uncrystallized form of the nonwoven fabric contained in the filter element from its quantity of heat of crystal melting is preferably 50 J/g or larger before steam heat treatment. This "value obtained by subtracting the quantity of heat of the uncrystallized form from the quantity of heat of crystal melting" is also an index that indicates the crystallinity of a resin. A larger value thereof means higher crystallinity of the resin. The further increased crystallinity further suppresses change in the physical properties (contraction, etc.) of the filter element between before and after steam heat treatment. Thus, the effect of enhancing removal performance for leukocytes and the like is obtained, as mentioned above.

The value obtained by subtracting the quantity of heat of the uncrystallized form from the quantity of heat of crystal melting is more preferably 55 J/g or larger, further preferably 60 J/g or larger, most preferably 65 J/g or larger.

In the present embodiment, the quantity of heat of the uncrystallized form and the quantity of heat of crystal melting are values measured as to the nonwoven fabric (fiber material) by differential scanning calorimetry (DSC). Such a measurement method will be described below.

From 3 to 4 mg of the nonwoven fabric (fiber material) is separated and loaded in an aluminum standard container. An initial heating curve (DSC curve) is measured at an initial temperature of 35°C at a heating rate of 10°C/min in an atmosphere of 50 mL/min nitrogen flow. An exothermic peak and a melting peak (endothermic peak) are detected from this initial heating curve (DSC curve). The values of quantity of heat (J) obtained from their respective peak areas are divided by the mass of the nonwoven fabric to calculate the quantity of heat of the uncrystallized form (J/g) and the quantity of heat of crystal melting (J/g).

For example, TA-60WS system manufactured by Shimadzu Corp. can be used as a measurement apparatus.

In the present embodiment, the X-ray crystallinity of the nonwoven fabric contained in the filter element is preferably 60 or larger before steam heat treatment. The further increased crystallinity of the filter element suppresses change in the physical properties (contraction, etc.) of the filter element between before and after steam heat treatment. Thus, the effect of enhancing removal performance for leukocytes and the like is obtained, as mentioned above.

The X-ray crystallinity is more preferably 63 or larger, further preferably 66 or larger.

In the present embodiment, the X-ray crystallinity is measured by an X-ray diffraction method.

The measurement can be performed by the following measurement steps 1) to 5) using an X-ray diffraction apparatus (e.g., MiniFlexII (Rigaku Corp., model 2005H301)) :

> 1) One nonwoven fabric (fiber material) having a size of 3 cm × 3 cm is loaded on a sample table.
> 2) The sample is assayed under the following conditions:
>
> > Scanning range: from 5° to 50°
> > Sampling width (width for data fetch): 0.02°
> > Scan speed: 2.0°/min
> > Voltage: 30 kV
> > Current: 15 mA
>
> 3) After the assay, data with peaks from an amorphous part and a crystalline part being separated from each other is obtained.
> 4) An amorphous peak area (Aa) and a total peak area (At) are determined from the data of the step 3). The data obtained in the step 3) is analyzed with, for example, analytical software (MDI JADE 7) to carry out an "automatic peak separation" function. As a result, the amorphous peak area (Aa) and the total peak area (At) are automatically calculated.
> 5) The crystallinity is calculated according to the following expression from the amorphous peak area (Aa) and the total peak area (At):

$$\mathrm{Crystallinity\ (\%)\ =\ (At\ -\ Aa)\ /\ At\ \times\ 100}$$

The nonwoven fabric whose quantity of heat of the uncrystallized form is 5 J/g or smaller, the nonwoven fabric whose value obtained by subtracting the quantity of heat of the uncrystallized form from the quantity of heat of crystal melting is 50 J/g or larger, and the nonwoven fabric having X-ray crystallinity of 60 or larger, before steam heat treatment can be easily produced, for example, by selecting a material or production conditions therefor as mentioned later.

In the present embodiment, the area contraction rate of the nonwoven fabric is preferably 10% or smaller, more preferably 3% or smaller, particularly preferably 2% or smaller, most preferably 1% or smaller. If the area contraction rate is larger than 10%, there is a tendency that not only is the pore size of the nonwoven fabric decreased by severe heat treatment such as high-temperature and high-pressure sterilization but the pore size becomes non-uniform to thereby increase clogging by blood cells and slow down processing speed. On the other hand, the area contraction rate of 10% or smaller is preferred because there is a tendency that the pore size is kept uniform even after sterilization treatment so that variation in processing speed can be prevented, and stable performance balance can be exerted.

In this respect, polybutylene terephthalate has a faster crystallization speed than that of other polyester fibers, for example, polyethylene terephthalate fibers. Therefore, its crystallinity is easily elevated. The resulting nonwoven fabric is less likely to contract in the planar direction even by severe steam heat treatment such as high-temperature and high-pressure sterilization (the area contraction rate is easily decreased) and can thus exert stable removal performance for leukocytes and the like and processing speed, irrespective of sterilization conditions.

The area contraction rate of the nonwoven fabric according to the present embodiment is calculated according to the following expression by accurately measuring the horizontal and vertical sizes of the nonwoven fabric (fiber material) cut into a square of approximately 20 cm × 20 cm, then performing heat treatment at 115°C for 240 minutes without fixing the nonwoven fabric with a pin or the like, and then measuring the horizontal and vertical sizes again:

```
Area contraction rate (%)

= (Vertical length (cm) of the nonwoven fabric before the heat treatment × Horizontal length (cm) of the

nonwoven fabric before the heat treatment - Vertical

length (cm) of the nonwoven fabric after the heat

treatment × Horizontal length (cm) of the nonwoven fabric after the heat treatment) / (Vertical length (cm) of the

nonwoven fabric before the heat treatment × Horizontal

length (cm) of the nonwoven fabric before the heat

treatment) × 100
```

It is preferred that the nonwoven fabric contained in the filter element should further have a basic nitrogen-containing functional group in a surface portion.

The surface portion of the nonwoven fabric refers to the entire portion, exposed to the outside world, of the nonwoven fabric. Specifically, the surface portion of the nonwoven fabric refers to the surface portion of the coat layer when the surface of the nonwoven fabric is coated with a coat layer containing a monomer and/or a polymer, etc., and refers to the portion, exposed to the outside world, of spun fibers present in the nonwoven fabric when no coat layer is formed on the fibers.

The phrase "having a basic nitrogen-containing functional group in a surface portion" of the nonwoven fabric means that the surface portion of the coat layer has a basic nitrogen-containing functional group when a coat layer is formed on the nonwoven fabric, and means that the portion, exposed to the outside world, of fibers present in the nonwoven fabric has a basic nitrogen-containing functional group when no coat layer is formed thereon.

The surface portion of the nonwoven fabric may further have a nonionic group.

In the filter element, the nonwoven fabric having a basic nitrogen-containing functional group in a surface portion produces the effect of enhancing the affinity of the nonwoven fabric for leukocytes in blood and thereby improving leukocyte removal performance.

Also, the nonwoven fabric containing a nonionic group in the surface portion can enhance the wettability of the nonwoven fabric surface for blood and improves the effective filtration area (area actually used in filtration) of the nonwoven fabric. The resulting nonwoven fabric is effective both for reduction in filtration time and for improvement in removal performance for leukocytes and the like.

The internal portion of the nonwoven fabric may or may not have a nonionic group or a basic nitrogen-containing functional group. The internal portion of the nonwoven fabric refers to the entire portion, unexposed to the outside world, of the nonwoven fabric. Specifically, the internal portion of the nonwoven fabric includes the inside of the fibers constituting the nonwoven fabric and also includes the coat layer-coated surface portion of the fibers unexposed to the outside world when the nonwoven fabric is coated with a coat layer.

Examples of methods for allowing the surface portion or the internal portion of the nonwoven fabric to contain a nonionic group or a basic nitrogen-containing functional group include a method of providing the nonwoven fabric with a coat layer using a coating material containing a monomer and/or a polymer containing the nonionic group or the basic nitrogen-containing functional group.

Alternatively, the resin constituting the fiber material may be mixed with a monomer and/or a polymer containing the nonionic group or the basic nitrogen-containing functional group when the fiber material of the nonwoven fabric is spun. This allows the nonionic group or the basic nitrogen-containing functional group to be contained and kneaded into the surface portion and the internal portion of the fiber material. In this way, the basic nitrogen-containing functional group or the like can be contained in the surface portion and the internal portion by one step, advantageously leading to the shortening of the production process, as compared with the method of forming a coat layer on the nonwoven fabric (mentioned above).

The ratio of the amount of substance of the basic nitrogen-containing functional group to the total amount of substance of the nonionic group and the basic nitrogen-containing functional group in the surface portion is preferably from 0.2 to 50.0% by mol, more preferably from 0.25 to 10% by mol, further preferably from 1 to 5% by mol, most preferably from 2 to 4% by mol. The content of the basic nitrogen-containing functional group can be measured by analysis based on NMR, IR, TOF-SIMS, or the like. The ratio between the basic nitrogen-containing functional group and the nonionic group can be set as described above to thereby secure stable wettability for blood and also efficiently remove leukocytes and the like while suppressing the unnecessary clogging of blood components such as platelets.

In the present embodiment, examples of the nonionic hydrophilic group include alkyl groups, alkoxy group, carbonyl groups, aldehyde groups, phenyl groups, amide groups, and hydroxyl groups.

In the present embodiment, examples of the basic nitrogen-containing functional group include amino groups represented by $-NH_2$, $-NHR_1$, $-NR_2R_3$, or $-N^+R^4R^5R^6$ ($R^1$, $R^2$, $R^3$, $R^4$, $R^5$, and $R^6$ each represent an alkyl group having from 1 to 3 carbon atoms).

In the present embodiment, the nonwoven fabric may be a fiber assembly (fiber material) itself (simple substance) or may have a coat layer on one or both of its surfaces. When the fiber material of the nonwoven fabric is coated with a coat layer, the surface $\zeta$ potential can be easily adjusted to 0 mV or larger by selecting a coating material, without drastically changing the method for producing the fiber material itself (and without consequently reducing filtration performance).

The coat layer preferably contains, for example, a copolymer having a monomer unit having the nonionic hydrophilic group and a monomer unit having the basic nitrogen-containing functional group. Use of the copolymer having the basic nitrogen-containing functional group is effective for imparting positive charge to the nonwoven fabric surface by coating treatment and is also effective for improving affinity for leukocytes.

Examples of the monomer unit having the nonionic hydrophilic group include units derived from 2-hydroxyethyl (meth)acrylate, 2-hydroxypropyl (meth)acrylate, vinyl alcohol, (meth)acrylamide, N-vinylpyrrolidone, and the like. Among these monomers, 2-hydroxyethyl (meth)acrylate is preferably used in view of easy availability, easy handling during polymerization, performance when blood flows, etc. The monomer unit of vinyl alcohol is usually formed by hydrolysis after polymerization of vinyl acetate.

Examples of the monomer unit having the basic nitrogen-containing functional group include units derived from: derivatives of (meth)acrylic acid such as diethylaminoethyl (meth)acrylate, dimethylaminoethyl (meth)acrylate, dimethylaminopropyl (meth)acrylate, and 3-dimethylamino-2-hydroxypropyl (meth)acrylate; styrene derivatives such as p-dimethylaminomethylstyrene and p-diethylaminoethylstyrene; vinyl derivatives of nitrogen-containing aromatic compounds such as 2-vinylpyridine, 4-vinylpyridine, and 4-vinylimidazole; derivatives in which the vinyl compounds described above are converted to quaternary ammonium salts with alkyl halides or the like; and the like. Among these monomers, diethylaminoethyl (meth)acrylate and dimethylaminoethyl (meth)acrylate are preferably used in view of easy availability, easy handling during polymerization, performance when blood flows, etc.

The mass of the coat layer is preferably from approximately 1.0 to 40.0 mg per 1 g in total of the masses of the fiber material and the coat layer. If the amount of coating is too large, uniform coating is disadvantageously difficult so that filtration performance may be deteriorated due to clogging by blood cells or drift of blood.

The mass of the coat layer can be calculated by, for example, the following procedures: the fiber material before carrying the coat layer is dried for 1 hour in a dryer set to 60°C, and then left for 1 hour or longer in a desiccator, followed by the measurement of the mass (A g). The fiber material carrying the coat layer is similarly dried for 1 hour in a dryer of 60°C and then left for 1 hour or longer in a desiccator, followed by the measurement of the mass (B g). The mass of the coat layer is calculated according to the following expression:

$$\text{Mass (mg/g) of the coat layer with respect to 1 g in}$$

$$\text{total of the fiber material and the coat layer} = (B - A)$$

$$\times\ 1000\ /\ B.$$

Examples of methods for forming a coat layer on the fiber material include, but are not limited to, a method of dipping the fiber material in a coating solution containing the monomer and/or the polymer (copolymer) and, if necessary, a solvent or the like, followed by the appropriate removal of the coating solution (dipping method), and a method of contacting the fiber material with a roll dipped in a coating solution to apply the coating solution thereto (transfer method).

In the case of preparing a filter by sandwiching and holding a filter element by two parts, outlet-side and inlet-side container materials, constituting a rigid container (e.g., as shown in Figures 1 and 2), when the filter element comprises a plurality of nonwoven fabrics, a nonwoven fabric having high crystallinity is used as a nonwoven fabric contacted with the outlet-side container material (nonwoven fabric disposed at the nearest position to the outlet-side container material) so that the filter element can be more strongly grasped by the holding part of the outlet-side container material after steam heat treatment. This suppresses a phenomenon in which blood leaks through the gaps between the holding parts and the filter element and directly runs into the outlet space from the inlet space without passing through the filter element (side leak phenomenon). Thus, the effect of improving removal performance for leukocytes and the like is obtained, and performance as a blood processing filter can be further improved.

Specifically, in the case of preparing a filter by sandwiching and holding a filter element by two parts, outlet-side and inlet-side container materials, constituting a rigid container, a nonwoven fabric contacted with the outlet-side container material among the nonwoven fabrics contained in the filter element preferably possesses the following (1) and more preferably possesses (2) and/or (3) in addition to (1):

(1) the quantity of heat of the uncrystallized form is 5 J/g or smaller before steam heat treatment,
(2) the value obtained by subtracting the quantity of heat of the uncrystallized form from the quantity of heat of crystal melting is 50 J/g or larger before steam heat treatment, and
(3) the X-ray crystallinity is 60 or larger before steam heat treatment.

In the case of preparing a filter by sandwiching and holding a filter element by two parts, outlet-side and inlet-side container materials, constituting a rigid container, the filter is excellent in terms of removal performance for leukocytes and the like after steam heat treatment if all of the nonwoven fabrics contained in the filter element have high crystallinity. However, the filter is inferior in terms of the ease of sandwiching and holding the filter element by the container materials or bonding the filter element with the container materials, due to the increased rebound strength of the filter element. Therefore, from the viewpoint of productivity in filter production, it is rather preferred that among the nonwoven fabrics contained in the filter element, a nonwoven fabric other than the nonwoven fabric contacted with the inlet-side container material or the outlet-side container material (or the nonwoven fabric contacted with the inlet-side container material or the outlet container material and a predetermined number (usually, from one to several) of nonwoven fabrics disposed adjacently thereto) should not have too high crystallinity.

When the filter element held in a rigid container comprises, for example, first and second nonwoven fabric layers (mentioned later) in this order from the inlet side, it is preferred that among a plurality of nonwoven fabrics contained in the second nonwoven fabric layer, a nonwoven fabric contacted with the outlet-side container material (and a predetermined number of nonwoven fabrics disposed adjacently thereto) should satisfy at least (1) described above, and one or some or all of the other nonwoven fabrics should not satisfy (1) described above or, if satisfying (1), should have a larger quantity of heat of the uncrystallized form before steam heat treatment than that of the nonwoven fabric contacted with the outlet-side container material, from the viewpoint of productivity in filter production.

The nonwoven fabric contained in the filter element of the present embodiment preferably has a formation index of 15 or larger and 70 or smaller corresponding to a thickness of 0.3 mm. If the formation index is larger than 70, the structure in the thickness direction of the nonwoven fabric is non-uniform relative to the filtration surface direction so that blood does not flow evenly in the nonwoven fabric. Therefore, there is a tendency that removal performance for leukocytes the like is reduced or a processing speed is slowed down. On the other hand, if the formation index is smaller than 15, clogging is more likely to occur due to a rise in liquid-flow resistance so that processing speed is slowed down. The formation index is more preferably 15 or larger and 65 or smaller, further preferably 15 or larger and 60 or smaller, particularly preferably 15 or larger and 50 or smaller, most preferably 15 or larger and 40 or smaller.

The formation index in the present embodiment is a value obtained by irradiating the nonwoven fabric (fiber material) with light from underneath, detecting the transmitted light with a charge-coupled device camera (hereinafter, abbreviated to a "CCD camera"), and multiplying the coefficient of variation (%) of the absorbance of the porous body (nonwoven fabric) detected by each pixel of the CCD camera by ten.

In the present embodiment, the formation index can be measured with, for example, a formation tester FMT-MIII (Nomura Shoji Co., Ltd.; manufactured in 2002; S/N: 130). The basic setting of the tester is not changed after shipment from the factory, and the measurement can be carried out such that the total number of pixels of a CCD camera is, for example, approximately 3400. Specifically, the measurement can be performed by adjusting the measurement size to 7 cm × 3 cm (one pixel size = 0.78 mm × 0.78 mm) such that the total number of pixels is approximately 3400. Alternatively, the measurement size may be changed according to the shape of a sample such that the total number of pixels is equal to 3400.

The formation index depends largely on the thickness of the nonwoven fabric. Therefore, the formation index corresponding to a thickness of 0.3 mm is calculated by the following method.

First, 3 nonwoven fabrics having a thickness of 0.3 mm or smaller are provided, and their respective formation indexes and thicknesses are measured. The thicknesses at arbitrary four points are measured at a measurement pressure of 0.4 N using a constant-pressure thickness meter (Ozaki Mfg. Co., Ltd., model FFA-12), and an average value thereof is used as the thickness of the nonwoven fabric. Next, two out of the 3 nonwoven fabrics thus assayed are stacked such that the thickness is 0.3 mm or larger. The formation index and the thickness of the two nonwoven fabrics in a stacked state are measured. After the completion of formation index measurement as to a total of 3 combinations, a linear regression equation of the thickness and the formation index is determined. The formation index corresponding to a thickness of 0.3 mm is determined from the equation.

The thickness of two nonwoven fabrics may fall short of 0.3 mm. In this case, a plurality of nonwoven fabrics are stacked such that the thickness of the stacked nonwoven fabrics is 0.3 mm or larger, followed by formation index measurement. Next, the formation index of a fewer number of nonwoven fabrics can be measured such that the thickness of the stacked nonwoven fabrics is 0.3 mm or smaller. The formation index is measured for all combinations of the nonwoven fabrics in which the thickness of the stacked nonwoven fabrics is 0.3 mm or smaller. A linear regression equation of the thickness and the formation index is determined. The formation index corresponding to a thickness of 0.3 mm can be determined from the equation.

The 3 or more nonwoven fabrics used in the formation index measurement are preferably cut out of a single filter element. They are usually nonwoven fabrics having substantially the same quality, i.e., nonwoven fabrics having the same physical properties (material, fiber diameter, bulk density, etc.). However, if the number of nonwoven fabrics having substantially the same quality necessary for the measurement cannot be obtained from a single filter element, the measurement can be performed by combining nonwoven fabrics from filter elements of the same type.

The specific method for calculating the formation index is, for example, also described in the paragraphs [0016] to [0018] of Japanese Patent No. 4134043.

The specific surface area of the nonwoven fabric contained in the filter element of the present embodiment is preferably 0.8 m²/g or larger and 3.2 m²/g or smaller. If the specific surface area is larger than 3.2 m²/g, there is a tendency that useful components such as plasma proteins are adsorbed onto the filter element during blood processing so that the recovery rate of the useful components is reduced. If the specific surface area is smaller than 0.8 m²/g, there is a tendency that removal performance for leukocytes and the like is reduced as compared with conventional filter elements because the amount of leukocytes and the like adsorbed is decreased.

The specific surface area of the nonwoven fabric is more preferably 1.0 m²/g or larger and 3.2 m²/g or smaller, further preferably 1.1 m²/g or larger and 2.9 m²/g or smaller, particularly preferably 1.2 m²/g or larger and 2.9 m²/g or smaller, most preferably 1.2 m²/g or larger and 2.6 m²/g or smaller.

The specific surface area according to the present embodiment refers to the surface area of the nonwoven fabric (fiber material) per unit mass and is a value measured by a BET adsorption method using nitrogen as an adsorption gas. The specific surface area can be measured using, for example, Tristar 3000 apparatus manufactured by Micromeritics Japan.

A larger specific surface area of the nonwoven fabric means that cells and plasma proteins, etc., can be adsorbed onto a larger area by blood processing using a filter element containing the nonwoven fabric having the same mass.

The airflow resistance of the nonwoven fabric contained in the filter element of the present embodiment is preferably 25 Pa·s·m/g or larger and 100 Pa·s·m/g or smaller, more preferably 30 Pa·s·m/g or larger and 90 Pa·s·m/g or smaller, further preferably 40 Pa·s·m/g or larger and 80 Pa·s·m/g or smaller.

If the airflow resistance is smaller than 25 Pa·s·m/g, there is a tendency that the number of contacts with leukocytes and the like is decreased so that the leukocytes and the like are difficult to capture. If the airflow resistance of the nonwoven fabric is larger than 100 Pa·s·m/g, there is a tendency that clogging by blood cells is increased so that processing speed is slowed down.

The airflow resistance of the nonwoven fabric of the embodiment is a value measured as differential pressure generated when air flows at a predetermined flow rate in the nonwoven fabric, and is a value obtained by placing the nonwoven fabric (fiber material) on a vent hole of an air permeability testing apparatus (e.g., manufactured by Kato Tech Co., Ltd., KES-F8-AP1), measuring a pressure drop (Pa·s/m) generated when air is allowed to flow at a flow rate of 4

mL/cm$^2$/sec for approximately 10 seconds, and further dividing the obtained pressure drop by the basis weight (g/m$^2$) of the nonwoven fabric. In this respect, the measurement is performed five times each with the cutout site changed, and an average value thereof is used as the airflow resistance.

Higher airflow resistance of the nonwoven fabric means that air is less likely to penetrate the nonwoven fabric, and the fibers constituting the nonwoven fabric are entangled in a dense or uniform state, and indicates that the nonwoven fabric has the property of hindering a blood product from flowing. On the other hand, lower airflow resistance of the nonwoven fabric means that the fibers constituting the nonwoven fabric are entangled in a coarse or non-uniform state, and indicates that the nonwoven fabric has the property of facilitating the flow of a blood product.

The mean flow pore size of the nonwoven fabric contained in the filter element of the present embodiment is preferably smaller than 8.0 $\mu$m. If the mean flow pore size is larger than 8.0 $\mu$m, there is a tendency that the number of contacts with leukocytes and the like is decreased so that the leukocytes and the like are difficult to capture. If the mean flow pore size is smaller than 1.0 $\mu$m, there is a tendency that clogging by blood cells is increased to thereby slow down processing speed. The mean flow pore size is more preferably 1.5 $\mu$m or larger and 7.5 $\mu$m smaller, further preferably 2.5 $\mu$m or larger and 7.0 $\mu$m or smaller, particularly preferably 3.5 $\mu$m or larger and 6.5 $\mu$m or smaller, most preferably 4.5 $\mu$m or larger and 6.5 $\mu$m or smaller.

The mean flow pore size of the nonwoven fabric (fiber material) of the present embodiment can be measured in accordance with ASTM F316-86 using Perm Porometer CFP-1200AEXS (automatic pore size distribution measurement system for porous materials) manufactured by Porous Materials, Inc. (PMI). A nonwoven fabric having a larger mean flow pore size facilitates the flow of a blood product, but reduces removal performance for leukocytes and the like. On the other hand, a nonwoven fabric having a smaller mean flow pore size improves removal performance for leukocytes and the like, but hinders a blood product from flowing and is also more likely to be clogged.

The filter element of the present embodiment may be constituted by one nonwoven fabric or may be constituted by a plurality of nonwoven fabrics. The filter element constituted by a plurality of nonwoven fabrics may be constituted by nonwoven fabrics of a single type or may be constituted by nonwoven fabrics of plural types. When the filter element is constituted by nonwoven fabrics of plural types, at least one of the nonwoven fabrics can have the preferred physical properties as to the nonwoven fabric described in the present specification. It is further preferred that all the nonwoven fabrics should have these physical properties.

As for the average fiber diameter of the nonwoven fabric, a nonwoven fabric layer comprising a fiber material having an average fiber diameter of from 0.3 to 3.0 $\mu$m is preferred from the viewpoint of the removal of leukocytes and the like.

When the filter element is constituted by nonwoven fabrics of plural types, it is preferred that the filter element should have a first nonwoven fabric layer which is disposed upstream and removes microaggregates, and a second nonwoven fabric layer which is disposed downstream of the first nonwoven fabric layer in order to remove leukocytes and the like. Each of the first and second nonwoven fabric layers may be one nonwoven fabric or may consist of a plurality of nonwoven fabrics. Each of the first and second nonwoven fabric layers each consisting of a plurality of nonwoven fabrics may be constituted by nonwoven fabrics of a single type or may be constituted by nonwoven fabrics of plural types.

The first nonwoven fabric layer disposed on the inlet side is preferably a nonwoven fabric layer comprising a fiber material having an average fiber diameter of from 3 to 60 $\mu$m, from the viewpoint of aggregate removal. The second nonwoven fabric layer is preferably a nonwoven fabric layer comprising a fiber material having an average fiber diameter of from 0.3 to 3.0 $\mu$m from the viewpoint of removing leukocytes and the like.

A post-filter layer may be further disposed, if necessary, downstream of the second nonwoven fabric layer.

The number of nonwoven fabrics constituting each nonwoven fabric layer can be appropriately selected in consideration of removal performance for leukocytes and the like required for the blood processing filter, a processing time, or balance thereof, etc., and may be, for example, one sheet for each.

The first nonwoven fabric layer of the filter element in this form is disposed upstream (on the inlet side) of the second nonwoven fabric layer, and the nonwoven fabric constituting the second nonwoven fabric layer has a smaller average fiber diameter than that of the nonwoven fabric constituting the first nonwoven fabric layer. Even if aggregates are formed in blood, the loose nonwoven fabric of the upstream (inlet-side) first nonwoven fabric layer thereby captures the aggregates to decrease the number of aggregates arriving at the fine nonwoven fabric of the downstream second nonwoven fabric layer. Thus, the clogging of the filter element by aggregates is suppressed. Particularly, the nonwoven fabric constituting the first nonwoven fabric layer has an average fiber diameter of from 3 to 60 $\mu$m and is effective for suppressing the clogging of the filter element. Also, the nonwoven fabric of the second nonwoven fabric layer has an average fiber diameter of smaller than 3 $\mu$m and can prevent reduction in removal performance for leukocytes and the like.

The average fiber diameter of the nonwoven fabric constituting the first nonwoven fabric layer is more preferably from 4 to 40 $\mu$m, further preferably from 30 to 40 $\mu$m and/or from 10 to 20 $\mu$m, because the clogging of the filter element can be suppressed more reliably. The average fiber diameter of the nonwoven fabric constituting the second nonwoven fabric layer is preferably 0.3 $\mu$m or larger because clogging by leukocytes and the like is prevented. Particularly, the average fiber diameter is more preferably from 0.5 to 2.5 $\mu$m from the viewpoint of removal performance for leukocytes and the like, etc.

A third nonwoven fabric layer consisting of a nonwoven fabric having an average fiber diameter of from 1.2 to 1.5 $\mu$m and/or from 0.9 to 1.2 $\mu$m may be further disposed for use downstream of the second nonwoven fabric layer.

The first nonwoven fabric layer containing a nonwoven fabric having a thick average fiber diameter and the second nonwoven fabric layer containing a nonwoven fabric having a thin average fiber diameter may be alternately arranged. In this case, it is preferred that the first nonwoven fabric layer, the second nonwoven fabric layer, the first nonwoven fabric layer, the second nonwoven fabric layer, ... should be arranged in this order from the inlet side, from the viewpoint of improvement in flowability by cascade structure formation.

The average fiber diameter according to the present embodiment refers to a value determined according to the following procedures:

A nonwoven fabric portion found to be substantially uniform is sampled at several sites from the nonwoven fabric actually constituting the filter element or one or two or more nonwoven fabrics having substantially the same quality thereas. The fibers in the nonwoven fabric samples are photographed under a scanning electron microscope such that their diameters are included therein.

The photographs are continuously taken until all the diameters of a total of 100 fibers are photographed. All the diameters of the photographed fibers are measured as to the photographs thus obtained. In this context, the diameter refers to the width of the fiber in the direction perpendicular to the fiber axis. The sum of all the measured diameters of the fibers is divided by the number of the fibers, and the obtained value is used as the average fiber diameter. However, when a plurality of fibers are overlapped so that the diameter of a fiber hidden behind another fiber cannot be measured, when a plurality of fibers are melted, for example, to form a thick fiber, when fibers significantly differing in diameter coexist, or when the boundary of the fibers is not clear due to the incorrect focus of a photograph, their data is deleted.

When the filter element contains a plurality of nonwoven fabrics and when the nonwoven fabrics evidently differ in measured fiber diameter, these nonwoven fabrics are of different types. Therefore, the interface between the different nonwoven fabrics is discovered, and their average fiber diameters are separately measured again. In this context, the phrase "evidently differ in average fiber diameter" refers to the case where a significant difference is statistically observed.

For a blood processing filter having a plate-like and flexible container, particularly, a post-filter layer is preferably disposed downstream of the second nonwoven fabric layer, because the flow of blood is prevented from being inhibited in such a way that filter element is pressed against the outlet-side container due to positive pressure on the inlet side generated during filtration and further, the outlet-side container is tightly contacted with the filter element due to negative pressure on the outlet side, and also because the weldability between the flexible container and the filter element is enhanced.

The post-filter layer can employ a filtration medium known in the art, such as a fibrous porous medium (e.g., nonwoven fabrics, woven fabrics, and meshes), or a porous body having three-dimensional network continuous pores. Examples of materials for these filtration media include polypropylene, polyethylene, styrene-isobutylene-styrene copolymers, polyurethane, and polyester. A post-filter layer made of a nonwoven fabric is preferred from the viewpoint of productivity and the welding strength of the blood processing filter. A post-filter layer having a plurality of protrusions by embossing or the like is particularly preferred because the flow of blood is rendered more uniform.

Each nonwoven fabric constituting the filter element may be modified at its surface by a technique known in the art, such as coating, chemical treatment, or radiation treatment, for the purpose of controlling selective separation properties for blood cells, surface hydrophilicity, etc.

For more reliably suppressing the clogging of the filter element, the bulk density of the nonwoven fabric constituting the first nonwoven fabric layer is preferably from 0.05 to 0.50 g/cm$^3$ and may be more preferably from 0.10 to 0.40 g/cm$^3$. If the bulk density of the nonwoven fabric of the first nonwoven fabric layer exceeds 0.50 g/cm$^3$, the nonwoven fabric might be clogged by the capture of aggregates or leukocytes and the like, resulting in a reduced filtration rate. On the other hand, if the bulk density falls below 0.05 g/cm$^3$, aggregate capture performance might be reduced so that the nonwoven fabric of the second nonwoven fabric layer is clogged, resulting in a reduced filtration rate. In addition, the mechanical strength of the nonwoven fabric may be reduced.

The "bulk density of the nonwoven fabric" is determined by cutting the nonwoven fabric (fiber material) at a site thought to be homogeneous into a size of 2.5 cm $\times$ 2.5 cm, measuring the basis weight (g/m$^2$) and the thickness (cm) by methods mentioned later, and dividing the basis weight by the thickness. In this respect, the measurement of the basis weight and the thickness is performed three times each with the cutout site changed, and an average value thereof is used as the bulk density.

The basis weight of the nonwoven fabric is determined by sampling a nonwoven fabric (fiber material) piece having a size of 2.5 cm $\times$ 2.5 cm from a site thought to be homogeneous, measuring the weight of the nonwoven fabric piece, and converting this weight to a mass per unit square meter. Also, the thickness of the nonwoven fabric is determined by sampling a nonwoven fabric (fiber material) piece having a size of 2.5 cm $\times$ 2.5 cm from a site thought to be homogeneous, and measuring the thickness of its center (one site) in a constant-pressure thickness meter. The load pressure of the constant-pressure thickness meter is set to 0.4 N, and the area of the measurement part is set to 2 $cm^2$.

The bulk density of the nonwoven fabric constituting the second nonwoven fabric layer is preferably from 0.05 to 0.50 $g/cm^3$, more preferably from 0.07 to 0.40 $g/cm^3$, further preferably from 0.10 to 0.30 $g/cm^3$. If the bulk density of the nonwoven fabric of the second nonwoven fabric layer is larger than 0.50 $g/cm^3$, there is a tendency that the flow resistance of the nonwoven fabric is increased, and clogging by blood cells is accordingly increased so that processing speed is slowed down. On the other hand, if the bulk density is smaller than 0.05 $g/cm^3$, there is a tendency that the number of contacts with leukocytes and the like is decreased so that the leukocytes and the like are difficult to capture. In addition, the mechanical strength of the nonwoven fabric may be reduced.

The nonwoven fabric more suitable for carrying out the present embodiment may be defined by a filling rate. The filling rate of the nonwoven fabric is calculated according to the following expression (10) by measuring the area, thickness, and mass of the nonwoven fabric (fiber material) cut into an arbitrary dimension and the specific gravity of the fiber material constituting the nonwoven fabric:

$$\text{Filling rate} = [\text{Mass (g) of the nonwoven fabric} /$$
$$(\text{Area } (cm^2) \text{ of the nonwoven fabric} \times \text{Thickness (cm) of}$$
$$\text{the nonwoven fabric})] / \text{Specific gravity } (g/cm^3) \text{ of the}$$
$$\text{fiber material constituting the nonwoven fabric} \cdots (10)$$

The filling rate of the nonwoven fabric contained in the filter element according to the present embodiment is preferably 0.04 or larger and 0.40 or smaller.

The filling rate of the first nonwoven fabric layer according to the embodiment mentioned above is more preferably from 0.08 to 0.30. On the other hand, the filling rate of the nonwoven fabric constituting the second nonwoven fabric layer is more preferably from 0.06 to 0.30, further preferably from 0.08 to 0.22.

If the filling rate of the first nonwoven fabric layer is larger than 0.40, there is a tendency that the flow resistance of the nonwoven fabric is increased by the capture of aggregates, leukocytes, and the like, and clogging by blood cells is accordingly increased so that processing speed is slowed down. On the other hand, if the filling rate is smaller than 0.04, aggregate capture performance might be reduced so that the nonwoven fabric of the second nonwoven fabric layer is clogged, resulting in a reduced filtration rate. In addition, the mechanical strength of the nonwoven fabric may be reduced.

If the filling rate of the nonwoven fabric of the second nonwoven fabric layer is larger than 0.40, there is a tendency that the flow resistance of the nonwoven fabric is increased, and clogging by blood cells is accordingly increased so that processing speed is slowed down. On the other hand, if the filling rate is smaller than 0.04, there is a tendency that the number of contacts with leukocytes and the like is decreased so that the leukocytes and the like are difficult to capture. In addition, the mechanical strength of the nonwoven fabric may be reduced.

In the present embodiment, examples of the fiber material for the nonwoven fabric contained in the filter element may include, but are not limited to, polymer materials such as polyester, polyamide, polyacrylonitrile, polymethyl methacrylate, polyethylene, and polypropylene. Also, metal fibers may be partially used. Use of fibers made of such a synthetic polymer material in the filter element can prevent the degeneration of blood. More preferably, the respective nonwoven fabrics of the first nonwoven fabric layer and the second nonwoven fabric layer having a stable fiber diameter can be obtained by adopting fibers containing polyester. Among others, PET or PBT is preferred because of having affinity for a blood product and stable wettability for blood.

In the present embodiment, the CWST (critical wetting surface tension) of the nonwoven fabric (when the nonwoven fabric has a coat layer, the nonwoven fabric is coated with a coat layer) contained in the filter element is preferably 70 dyn/cm or larger, more preferably 85 dyn/cm or larger, further preferably 95 dyn/cm or larger. The nonwoven fabric having such a critical wetting surface tension secures stable wettability for blood and is thereby capable of efficiently removing leukocytes and the like while allowing platelets in a blood product to pass therethrough.

The CWST refers to a value determined according to the following method: aqueous solutions of sodium hydroxide, calcium chloride, sodium nitrate, acetic acid, or ethanol differing in concentration are prepared such that the surface tension varies by from 2 to 4 dyn/cm. The surface tension (dyn/cm) of each aqueous solution thus obtained is from 94 to 115 for the aqueous sodium hydroxide solutions, from 90 to 94 for the aqueous calcium chloride solutions, from 75 to 87 for the aqueous sodium nitrate solutions, 72.4 for pure water, from 38 to 69 for the aqueous acetic acid solutions, and from 22 to 35 for the aqueous ethanol solutions ("Kagaku Binran (Handbook of Chemistry in English), Basics II", revised 2nd edition., edited by The Chemical Society of Japan, Maruzen Publishing Co., Ltd., 1975, p. 164). Ten drops each of the thus-obtained aqueous solutions differing in surface tension by from 2 to 4 dyn/cm are placed on the nonwoven fabric in the ascending order of the surface tension, and left for 10 minutes. The case where 9 or more out of the 10 drops thus left for 10 minutes are absorbed by the nonwoven fabric is defined as a wet state, and the case where less than 9 out of the 10 drops are absorbed thereby is defined as a non-wet state. In this way, the liquids are assayed in the ascending order of the surface tension on the nonwoven fabric. During this assay, the wet state shifts to the non-wet state. In this respect, the CWST value of the nonwoven fabric is defined as an average value of the surface tension value of the last liquid for which the wet state is observed and the surface tension value of the first liquid for which the non-wet state is observed. For example, the CWST value of the nonwoven fabric that is wet by a liquid having a surface tension of 64 dyn/cm and is non-wet by a liquid having a surface tension of 66 dyn/cm is 65 dyn/cm.

The nonwoven fabric (fiber material) contained in the filter element of the present embodiment is not limited by its production method, and can be produced by any of wet and dry methods. In the present embodiment, the nonwoven fabric is particularly preferably produced by a melt blown method because a nonwoven fabric having the optimum formation index and average fiber diameter is stably obtained.

One example of the melt blown method will be described as the method for producing the nonwoven fabric (fiber material) used in the present embodiment. In the melt blown method, a molten polymer fluid obtained by melting in an extruder is filtered through an appropriate filter, then introduced to a molten polymer inlet of a melt blown die, and then discharged from an orifice nozzle. At the same time therewith, a heated gas introduced to a heated gas inlet is introduced to a heated gas ejection slit formed from the melt blown die and a lip, and ejected therefrom so that the discharged molten polymer is attenuated to form ultrathin fibers. The formed ultrathin fibers are laminated to thereby obtain a nonwoven fabric. The nonwoven fabric can be further heat-treated using a heat suction drum, a hot plate, hot water, a hot air heater, etc. to obtain a nonwoven fabric having the desired crystallinity.

For the preparation of the nonwoven fabric by a melt blown method, a nonwoven fabric having a smaller average fiber diameter can be obtained by applying a higher quantity of heat to the molten polymer. This is probably because the application of the quantity of heat decreases the viscosity of the polymer and facilitates attenuating the molten polymer. On the other hand, the application of too high a quantity of heat degrades the polymer itself and increases a carboxyl group equivalent. It is therefore desirable to adjust the quantity of heat according to the properties of the polymer.

For producing the nonwoven fabric (fiber material) having a small average fiber diameter and a carboxyl group equivalent of, for example, from 20 to 140 ($\mu$eq/g), the temperature of polymer melting is preferably a temperature of from [melting point of the polymer + 20]°C to [melting point of the polymer + 150]°C, more preferably from [melting point of the polymer + 40]°C to [melting point of the polymer + 120]°C.

For applying thereto a necessary and sufficient quantity of heat for heat treatment after nonwoven fabric formation, it is also desirable to adjust the heating temperature and time according to the properties of the polymer.

For producing the nonwoven fabric having high crystallinity and a carboxyl group equivalent of, for example, from 20 to 140 ($\mu$eq/g), the heating temperature is preferably a temperature equal to or higher than [melting point of the polymer - 120]°C, more preferably from [melting point of the polymer - 120]°C to [melting point of the polymer - 60]°C. The heating time varies depending on the heating temperature and is preferably at least 3 seconds or longer, more preferably 10 seconds or longer, further preferably 20 seconds or longer, particularly preferably 30 seconds or longer.

If the heating temperature is lower than [melting point of the polymer - 120]°C or if the heating time is shorter than 3 seconds, the crystallinity of the polymer to be satisfied tends to be difficult to obtain. As one example, a sufficient quantity of heat suitable for the present embodiment can be applied thereto by allowing the polybutylene terephthalate nonwoven fabric after spinning to stay in dry air of 140°C for 120 seconds.

In the present embodiment, the method for adjusting the carboxyl group equivalent of the nonwoven fabric to 20 to 140 ($\mu$eq/g) is not limited.

When the nonwoven fabric is made of the fiber material alone, the fiber material can be spun such that its carboxyl group equivalent is 20 to 140 ($\mu$eq/g), as mentioned above.

When the nonwoven fabric has a coat layer, a fiber material having an appropriate carboxyl group equivalent can be spun according to the amount of a carboxyl group contained in a coating material. For example, an appropriate combination of the coating material and the fiber material can be determined by repetitively (approximately several times) prototyping the fiber material with its carboxyl group equivalent changed while measuring the carboxyl group equivalent of the resulting nonwoven fabric.

In the present embodiment, the filter element can be housed in a container to thereby prepare a blood processing filter.

The material for the container which houses the filter element may be any of rigid resins and flexible resins. Examples of the rigid resin material include phenol resin, acrylic resin, epoxy resin, formaldehyde resin, urea resin, silicon resin, ABS resin, nylon, polyurethane, polycarbonate, vinyl chloride, polyethylene, polypropylene, polyester, and styrenebutadiene copolymers.

The flexible resin material for the container is preferably similar in thermal and electrical properties to the filter element. Examples of suitable materials include: thermoplastic elastomers such as soft polyvinyl chloride, polyurethane, ethylene-vinyl acetate copolymers, polyolefins such as polyethylene and polypropylene, hydrogenation products of styrenebutadiene-styrene copolymers, and styrene-isoprene-styrene copolymers or hydrogenation products thereof; and mixtures of the thermoplastic elastomers with softening agents such as polyolefins and ethylene-ethyl acrylate. The material is preferably soft vinyl chloride, polyurethane, an ethylene-vinyl acetate copolymer, a polyolefin, or a thermoplastic elastomer composed mainly of any of them, more preferably soft vinyl chloride or a polyolefin.

The shape of the container is not particularly limited as long as the shape has an inlet for a liquid to be processed (leukocyte-containing liquid) and an outlet for a processed (leukocyte-free) liquid. The shape is preferably adapted to the shape of the filter element.

When the filter element is, for example, plate-like, the container can have a flat shape consisting of a polygon such as a tetragon or a hexagon, a circle, an ellipse, or the like according to the plate-like shape. More specific examples thereof include configuration in which, as shown in Figure 1 or 2, the container 1 is constituted by an inlet-side container material having the first port 3 as a liquid inlet/outlet and an outlet-side container material having the second port 4 as a liquid inlet/outlet, and both the container materials sandwich the filter element 5 either directly or via a support such that the inside of the filter is divided into two rooms to form the flat blood processing filter 10.

As another example, when the filter element is cylindrical, it is preferred that the container should also be cylindrical. More specifically, the container is constituted by a tubular barrel which houses the filter element, an inlet-side header having a liquid inlet, and an outlet-side header having a liquid outlet, and preferably has a shape in which the inside of the container is divided into two rooms by potting such that a liquid introduced from the inlet flows from the outer periphery to the inner periphery (or from the inner periphery to the outer periphery) of the cylindrical filter, to form the cylindrical blood processing filter.

Next, a leukocyte removal method using the blood processing filter of the present embodiment will be described.

The leukocyte removal method of the present embodiment comprises the step of allowing a leukocyte-containing liquid to pass through a blood processing filter, to remove leukocytes from the leukocyte-containing liquid.

In this context, the leukocyte-containing liquid is a generic name for body fluids and synthetic blood containing leukocytes, and is specifically whole blood and a liquid consisting of a single or plural types of blood components obtained by preparation from whole blood, such as whole blood, a concentrated red cell solution, a washed red cell suspension, a thawed red cell concentrate, synthetic blood, platelet-poor plasma (PPP), platelet-rich plasma (PRP), plasma, frozen plasma, a platelet concentrate, and buffy coat (BC); a solution in which the liquid is supplemented with an anticoagulant, a preservative solution, or the like; or a whole blood product, a red cell product, a platelet product, or a plasma product and the like.

Also, a liquid obtained by processing the liquid mentioned above by the method of the present embodiment is referred to as a leukocyte-free liquid.

Hereinafter, one mode of a method for preparing each blood product by removing leukocytes by the leukocyte removal method will be described.

(Preparation of leukocyte-free whole blood product)

The leukocyte-free whole blood product can be obtained by providing a whole blood product by the addition of, for example, a preservative solution or an anticoagulant, such as citrate phosphate dextrose (CPD), citrate phosphate dextrose adenine-1 (CPDA-1), citrate phosphate-2-dextrose (CP2D), acid citrate dextrose formula-A (ACD-A), acid citrate dextrose formula-B (ACD-B), or heparin, to collected whole blood, and then removing leukocytes from the whole blood product using the blood processing filter of the present embodiment.

In the preparation of the leukocyte-free whole blood product, in the case of leukocyte removal before preservation, the whole blood preserved at room temperature or under refrigeration can be subjected to leukocyte removal using the blood processing filter at room temperature or under refrigeration preferably within 72 hours, more preferably within 24 hours, particularly preferably within 12 hours, most preferably within 8 hours after blood collection to obtain the leukocyte-free whole blood product. In the case of leukocyte removal after preservation, leukocytes can be removed from the whole blood preserved at room temperature, under refrigeration, or under freezing, preferably within 24 hours before use, using the blood processing filter to obtain the leukocyte-free whole blood product.

(Preparation of leukocyte-free red cell product)

A preservative solution or an anticoagulant, such as CPD, CPDA-1, CP2D, ACD-A, ACD-B, or heparin, is added to collected whole blood. A separation method for each blood component includes the case of performing centrifugation after removal of leukocytes from the whole blood, and the case of removing leukocytes from red cells or red cells and BC after centrifugation of the whole blood.

In the case of performing centrifugation after removal of leukocytes from the whole blood, the leukocyte-free red cell product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. After addition of a preservative solution such as SAGM, AS-1, AS-3, AS-5, or MAP, if necessary, to red cells separated from the whole blood or red cells containing BC, leukocytes can be removed from the red cells using the leukocyte removal filter to obtain the leukocyte-free red cell product.

In the preparation of the leukocyte-free red cell product, the whole blood preserved at room temperature or under refrigeration can be centrifuged preferably within 72 hours, more preferably within 48 hours, particularly preferably within 24 hours, most preferably within 12 hours after blood collection.

In the case of leukocyte removal before preservation, leukocytes can be removed from the red cell product preserved at room temperature or under refrigeration, preferably within 120 hours, more preferably within 72 hours, particularly preferably within 24 hours, most preferably within 12 hours after blood collection, using the blood processing filter at room temperature or under refrigeration to obtain the leukocyte-free red cell product. In the case of leukocyte removal after preservation, leukocytes can be removed from the red cell product preserved at room temperature, under refrigeration, or under freezing, preferably within 24 hours before use, using the blood processing filter to obtain the leukocyte-free red cell product.

(Preparation of leukocyte-free platelet product)

A preservative solution or an anticoagulant, such as CPD, CPDA-1, CP2D, ACD-A, ACD-B, or heparin, is added to collected whole blood.

A separation method for each blood component includes the case of performing centrifugation after removal of leukocytes from the whole blood, and the case of removing leukocytes from PRP or platelet after centrifugation of the whole blood.

In the case of performing centrifugation after removal of leukocytes from the whole blood, the leukocyte-free platelet product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. Under the soft spin conditions, leukocytes are removed from PRP separated from the whole blood with the blood processing filter, and then, the leukocyte-free platelet product is obtained by centrifugation, or platelet and PPP are obtained by centrifuging PRP, and then, leukocytes can be removed with the blood processing filter to obtain the leukocyte-free platelet product. Under the hard spin conditions, a pool of one unit or several to dozen units of BC separated from the whole blood is supplemented, if necessary, with a preservative solution, plasma, or the like, and centrifuged to obtain platelet, and leukocytes can be removed from the obtained platelet with the blood processing filter to obtain the leukocyte-free platelet product.

In the preparation of the leukocyte-free platelet product, the whole blood preserved at room temperature is centrifuged preferably within 24 hours, more preferably within 12 hours, particularly preferably within 8 hours after blood collection. In the case of leukocyte removal before preservation, leukocytes can be removed from the platelet product preserved at room temperature, preferably within 120 hours, more preferably within 72 hours, particularly preferably within 24 hours, most preferably within 12 hours after blood collection, using the blood processing filter at room temperature to obtain the leukocyte-free platelet product. In the case of leukocyte removal after preservation, leukocytes can be removed from the platelet product preserved at room temperature, under refrigeration, or under freezing, preferably within 24 hours before use, using the blood processing filter to obtain the leukocyte-free platelet product.

(Preparation of leukocyte-free plasma product)

A preservative solution or an anticoagulant, such as CPD, CPDA-1, CP2D, ACD-A, ACD-B, or heparin, is added to collected whole blood.

A separation method for each blood component includes the case of performing centrifugation after removal of leukocytes from the whole blood, and the case of removing leukocytes from PPP or PRP after centrifugation of the whole blood.

In the case of performing centrifugation after removal of leukocytes from the whole blood, the leukocyte-free plasma product can be obtained by centrifuging the leukocyte-free whole blood.

In the case of centrifuging the whole blood before leukocyte removal, the centrifugation conditions are divided into two types: soft spin conditions where the whole blood is separated into red cells and PRP, and hard spin conditions where the whole blood is separated into red cells, BC, and PPP. Under the soft spin conditions, leukocytes are removed from PRP with the blood processing filter, and then, the leukocyte-free plasma product is obtained by centrifugation, or PRP is centrifuged into PPP and platelet, and then, leukocytes can be removed with the blood processing filter to obtain the leukocyte-free plasma product. Under the hard spin conditions, leukocytes can be removed from PPP with the blood processing filter to obtain the leukocyte-free plasma product.

In the preparation of the leukocyte-free plasma product, the whole blood preserved at room temperature or under refrigeration can be centrifuged preferably within 72 hours, more preferably within 48 hours, particularly preferably within 24 hours, most preferably within 12 hours after blood collection. Leukocytes can be removed from the plasma product preserved at room temperature or under refrigeration, preferably within 120 hours, more preferably within 72 hours, particularly preferably within 24 hours, most preferably within 12 hours after blood collection, using the blood processing filter at room temperature or under refrigeration to obtain the leukocyte-free plasma product. In the case of leukocyte removal after preservation, leukocytes can be removed from the plasma product preserved at room temperature, under refrigeration, or under freezing, preferably within 24 hours before use, using the blood processing filter to obtain the leukocyte-free plasma product.

Any mode such as a mode of collecting blood with a blood collection needle connected with a container for whole blood, and connecting the container containing whole blood or blood components after centrifugation with the blood processing filter, followed by leukocyte removal, a mode of collecting blood using a circuit in which at least a blood collection needle, a blood container, and the blood processing filter are sterilely connected, and performing leukocyte removal before centrifugation or after centrifugation, or a mode of connecting the blood processing filter with a container containing blood components obtained in an automatic blood collection apparatus or using the blood processing filter connected in advance with the container to perform leukocyte removal may be used as a mode from blood collection to the preparation of a leukocyte-free blood product, though the present embodiment is not limited by these modes. Alternatively, the leukocyte-free red cell product, the leukocyte-free platelet product, or the leukocyte-free plasma product may be obtained by centrifuging whole blood into each component in an automatic blood component collection apparatus, if necessary adding a preservative solution, and immediately thereafter allowing any of red cells, BC-containing red cells, BC, platelet, PRP, and PPP to pass through the blood processing filter to remove leukocytes.

The method of the present embodiment has higher leukocyte removal performance for all types of blood described above and is effective for shortening a processing time without causing clogging. The method of the present embodiment is particularly suitable for suppressing the production of bradykinin in the processing of whole blood, platelet-poor plasma (PPP), or platelet-rich plasma (PRP), which contains plasma protein components at a high concentration and is prone to produce bradykinin.

In this context, the amount of bradykinin produced (bradykinin production rate) by the contact of the blood processing filter with blood is, in terms of a bradykinin concentration in processed blood, preferably 1 or more times and less than 100 times, more preferably 1 or more times and less than 80 times, most preferably 1 or more times and less than 60 times of the concentration before the processing.

The bradykinin concentration can be conveniently measured by a method known in the art, such as radioimmunoassay or enzyme immunoassay. Specifically, blood is centrifuged at a centrifugal force of $3000 \times g$ at room temperature for 10 minutes before and after blood processing. Then, the supernatant fraction is collected and measured by radioimmunoassay.

A complement may be activated during processing of a blood product containing plasma proteins through a blood processing filter. The activation concentration value of C3a, which is easily activated, can be used as an index for complement activation. This permits favorable evaluation of biocompatibility. In this context, the concentration of C3a to be activated is preferably lower. The value of C3a is preferably 0.5 or more times and less than 10 times that before blood processing. More preferably, the value of C3a is 0.5 or more times and less than 8 times, most preferably 0.5 or more times and less than 6 times that before blood processing.

The concentration of C3a can be measured by a method known in the art, such as radioimmunoassay 2-antibody method (Japanese Journal of Clinical Medicine, Vol. 53, 1995, extra number (last volume)).

In the present embodiment, the leukocyte removal may be performed by dropping leukocyte-containing blood from a container containing the leukocyte-containing liquid located at a position higher than the blood processing filter to flow into the blood processing filter via a tube, or may be performed by allowing the leukocyte-containing blood to flow by increasing pressure from the inlet side of the blood processing filter and/or reducing pressure from the outlet side of the blood processing filter using means such as a pump.

Hereinafter, a leukocyte removal method using the blood processing filter for extracorporeal circulation therapy will be described.

The inside of the blood processing filter is primed with physiological saline or the like, which is then replaced with a solution containing an anticoagulant such as heparin, nafamostat mesilate, ACD-A, or ACD-B. While the anticoagulant is added to blood diverted outside the body, the blood is injected into the inlet of the blood processing filter from a circuit connected with a human at a flow rate of from 10 to 200 mL/min, and leukocytes can be removed with the blood processing filter.

In the initial period of leukocyte removal (throughput: from 0 to 0.5 L), the flow rate is preferably from 10 to 50 mL/min, more preferably from 20 to 40 mL/min. After the initial period of leukocyte removal (throughput: from 0.2 to 12 L), the blood is preferably processed at a flow rate of from 30 to 120 mL/min, more preferably from 40 to 100 mL/min, particularly preferably from 40 to 60 mL/min. It is preferred to substitute the inside of the blood processing filter with physiological saline or the like after the leukocyte removal to return the blood, because the blood within the blood processing filter is not wasted.

Examples

Hereinafter, the present invention will be described with reference to Examples.

[Example 1]

(Preparation of nonwoven fabric)

Polybutylene terephthalate (hereinafter, abbreviated to PBT) was spun by the melt blown method to form a fiber assembly, followed by the heat treatment of the obtained fiber assembly at 140°C for 120 seconds to prepare a fiber material. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 122 $\mu$eq/g.
The obtained fiber material was coated with a hydrophilic polymer by a method described below to obtain a nonwoven fabric. The hydrophilic polymer used contained no carboxyl group, and the carboxyl group equivalent of the nonwoven fabric thus coated was 122 $\mu$eq/g which was the same as that of the fiber material.
A copolymer of 2-hydroxyethyl methacrylate (hereinafter, abbreviated to HEMA) and diethylaminoethyl methacrylate (hereinafter, abbreviated to DEAMA) was synthesized by usual solution radical polymerization. The polymerization reaction was performed at a monomer concentration of 1 mol/L in ethanol at 60°C for 8 hours in the presence of 1/200 mol of azoisobutyronitrile (AIBN) as an initiator. The fiber material was dipped in the ethanol solution of the formed hydrophilic polymer. The absorbed redundant polymer solution was squeezed out of the fiber material removed from the polymer solution, and the polymer solution was dried off while dry air was sent, to form a coat layer covering the surface of the fiber material.
The ratio of the amount of substance of the basic nitrogen-containing functional group to the total amount of substance of the nonionic group and the basic nitrogen-containing functional group in the surface portion (surface portion of the coat layer) of the obtained nonwoven fabric was 3.0% by mol. The mass of the coat layer per g of the nonwoven fabric was 9.0 mg/g (fiber material + coat layer). The CWST value was 100 dyn/cm. Furthermore, the surface $\zeta$ potential of the nonwoven fabric thus coated was 36 mV.

(Preparation of filter for blood processing)

A rigid container having an effective filtration area of 45 cm$^2$ was packed with 64 sheets of the obtained nonwoven fabric provided with the coat layer, and ultrasonically welded with this filter element to prepare a filter.
This filter was steam heat-treated at 115°C for 240 minutes and then vacuum-dried at 40°C for 15 hours or longer to prepare a steam heat-treated filter.

(Leukocyte removal performance evaluation)

Next, a testing method to evaluate leukocyte removal performance will be described.
The blood used in evaluation was whole blood prepared by adding 70 mL of an anticoagulant CPD solution to 500 mL of blood immediately after blood collection, mixing them, and leaving the mixture standing for 2 hours. Hereinafter, this blood prepared for blood evaluation is referred to as pre-filtration blood.
A blood bag packed with the pre-filtration blood was connected with the inlet of the steam heat-treated filter through a 40 cm polyvinyl chloride tube having an inside diameter of 3 mm and an outside diameter of 4.2 mm. Further, a blood bag for recovery was similarly connected with the outlet of the filter through a 60 cm polyvinyl chloride tube having an inside diameter of 3 mm and an outside diameter of 4.2 mm. Then, the pre-filtration blood was dropped 100 cm from the bottom of the blood bag packed with the pre-filtration blood to flow into the filter. The filtration time was measured until the amount of the blood flowing into the recovery bag became 0.5 g/min.
3 mL of blood (hereinafter, referred to as post-filtration blood) was further recovered from the recovery bag. The leukocyte removal performance was evaluated by determining a leukocyte residual rate. The leukocyte residual rate was calculated according to the following expression by measuring the number of leukocytes in the pre-filtration blood and the post-filtration blood using flow cytometry (apparatus: FACSCanto manufactured by Becton, Dickinson and Company):

$$\text{Leukocyte residual rate}$$

$$= [\text{Leukocyte concentration (number/}\mu\text{L) (post-filtration}$$

$$\text{blood)}] / [\text{Leukocyte concentration (number/}\mu\text{L) (pre-}$$

$$\text{filtration blood)}].$$

The measurement of the number of leukocytes was performed by sampling 100 $\mu$l of each blood and using Leucocount kit (Becton, Dickinson and Company, Japan) containing beads.

(Blood quality evaluation)

The respective bradykinin concentrations and C3a concentrations of pre-filtration blood and post-filtration blood were further measured, and the bradykinin production rate and the C3a activation rate were calculated according to the following expressions:

$$\text{Bradykinin production rate} = \text{Bradykinin}$$

$$\text{concentration after filtration} / \text{Bradykinin concentration}$$

$$\text{before filtration}$$

$$\text{C3a activation rate} = \text{C3a concentration after}$$

$$\text{filtration} / \text{C3a concentration before filtration}$$

In the case of conducting evaluation with the filter shape described above (64 sheets of the nonwoven fabric, effective filtration area: 45 cm$^2$), a leukocyte removal filter element that can achieve a filtration time of 30 minutes or shorter and a leukocyte residual rate of $10.0 \times 10^{-3}$ or less is regarded as being practically desirable. Specifically, at a leukocyte residual rate of $10^{-4}$ or less, the number of residual leukocytes is close to the measurement limit. Therefore, here, the filter shape conditions were set as described above so as to attain the leukocyte residual rate of $10^{-4}$ or more. A filter element having performance that satisfies the filtration time of 30 minutes or shorter and the leukocyte residual rate of $10.0 \times 10^{-3}$ or less under this conditions can be designed suitably for actual use and thereby produced as a filter that can achieve a leukocyte residual rate of from $10^{-4}$ to $10^{-6}$ or less necessary for preventing severe adverse reactions.

As a result, the leukocyte residual rate was $6.2 \times 10^{-3}$, and the filtration time was 9.0 minutes, demonstrating low blood process pressure and high leukocyte removal performance. Also, the bradykinin production rate was 3.1, and the C3a activation rate was 1.8, demonstrating that the post-filtration blood has good blood quality.

[Example 2]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 131 $\mu$eq/g.

The obtained fiber material was subjected to polymer coating treatment in the same manner as in Example 1. The carboxyl group equivalent of the nonwoven fabric thus polymer-coated was 131 $\mu$eq/g. The mass of the coat layer per g of the nonwoven fabric was 26.2 mg/g (fiber material + coat layer). The surface $\zeta$ potential of the nonwoven fabric thus coated was 96 mV.

A filter was prepared in the same way as in Example 1 using the nonwoven fabric thus polymer-coated and subjected to the blood test.

As a result, the leukocyte residual rate was $0.7 \times 10^{-3}$, and the filtration time was 21.0 minutes, demonstrating high leukocyte removal performance and a short filtration time. Also, the bradykinin production rate was 1.7, and the C3a activation rate was 5.3, demonstrating that the post-filtration blood has good blood quality.

[Example 3]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 127 $\mu$eq/g.

The obtained fiber material was subjected to polymer coating treatment in the same manner as in Example 1. The carboxyl group equivalent of the nonwoven fabric thus polymer-coated was 127 $\mu$eq/g. The mass of the coat layer per g of the nonwoven fabric was 38.7 mg/g (fiber material + coat layer). The surface $\zeta$ potential of the nonwoven fabric thus coated was 123 mV.

A filter was prepared in the same way as in Example 1 using the nonwoven fabric thus polymer-coated and subjected to the blood test.

As a result, the leukocyte residual rate was 0.2 $\times$ 10$^{-3}$, and the filtration time was 29.6 minutes, demonstrating high leukocyte removal performance and a short filtration time. Also, the bradykinin production rate was 1.2, and the C3a activation rate was 9.6, demonstrating that the post-filtration blood has good blood quality.

[Example 4]

A fiber material was prepared by the method of spinning a fiber assembly made of PET fibers by the melt blown method, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 26 $\mu$eq/g.

The obtained fiber material was subjected to polymer coating treatment in the same manner as in Example 1. The carboxyl group equivalent of the nonwoven fabric thus polymer-coated was 26 $\mu$eq/g. The mass of the coat layer per g of the nonwoven fabric was 4.2 mg/g (fiber material + coat layer). The surface $\zeta$ potential of the nonwoven fabric thus coated was 23 mV.

A filter was prepared in the same way as in Example 1 using the nonwoven fabric thus polymer-coated and subjected to the blood test.

As a result, the leukocyte residual rate was 7.6 $\times$ 10$^{-3}$, and the filtration time was 7.4 minutes, demonstrating high leukocyte removal performance and a short filtration time. Also, the bradykinin production rate was 11.4, and the C3a activation rate was 1.5, demonstrating that the post-filtration blood has good blood quality.

[Example 5]

A fiber material was prepared by the method of spinning a fiber assembly made of PET fibers, followed by the heat treatment of the fiber assembly thus spun in the same way as in Example 4. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 21 $\mu$eq/g.

The obtained fiber material was subjected to polymer coating treatment in the same manner as in Example 1. The carboxyl group equivalent of the nonwoven fabric thus polymer-coated was 21 $\mu$eq/g. The mass of the coat layer per g of the nonwoven fabric was 13.8 mg/g (fiber material + coat layer). The surface $\zeta$ potential of the nonwoven fabric thus coated was 99 mV.

A filter was prepared in the same way as in Example 1 using the nonwoven fabric thus polymer-coated and subjected to the blood test.

As a result, the leukocyte residual rate was 1.2 $\times$ 10$^{-3}$, and the filtration time was 20.2 minutes, demonstrating high leukocyte removal performance and a short filtration time. Also, the bradykinin production rate was 3.7, and the C3a activation rate was 4.4, demonstrating that the post-filtration blood has good blood quality.

[Example 6]

A fiber material was prepared by the method of spinning a fiber assembly made of PET fibers, followed by the heat treatment of the fiber assembly thus spun in the same way as in Example 4. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 28 $\mu$eq/g.

The obtained fiber material was subjected to polymer coating treatment in the same manner as in Example 1. The carboxyl group equivalent of the nonwoven fabric thus polymer-coated was 28 $\mu$eq/g. The mass of the coat layer per g of the nonwoven fabric was 29.7 mg/g (fiber material + coat layer). The surface $\zeta$ potential of the nonwoven fabric thus coated was 132 mV.

A filter was prepared in the same way as in Example 1 using the nonwoven fabric thus polymer-coated and subjected to the blood test.

As a result, the leukocyte residual rate was $0.4 \times 10^{-3}$, and the filtration time was 28.7 minutes, demonstrating high leukocyte removal performance and a short filtration time. Also, the bradykinin production rate was 2.1, and the C3a activation rate was 8.7, demonstrating that the post-filtration blood has good blood quality.

[Example 7]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m², a thickness of 0.13 mm, a filling rate of 0.12, an average fiber diameter of 1.5 $\mu$m, and a carboxyl group equivalent of 27 $\mu$eq/g.

The obtained fiber material was subjected to polymer coating treatment in the same manner as in Example 1. The carboxyl group equivalent of the nonwoven fabric thus polymer-coated was 27 $\mu$eq/g. The mass of the coat layer per g of the nonwoven fabric was 5.3 mg/g (fiber material + coat layer). The surface $\zeta$ potential of the nonwoven fabric thus coated was 42 mV.

A filter was prepared in the same way as in Example 1 using the nonwoven fabric thus polymer-coated and subjected to the blood test.

As a result, the leukocyte residual rate was $9.8 \times 10^{-3}$, and the filtration time was 8.5 minutes, demonstrating high leukocyte removal performance and a short filtration time. Also, the bradykinin production rate was 1.3, and the C3a activation rate was 1.2, demonstrating that the post-filtration blood has good blood quality.

[Example 8]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m², a thickness of 0.13 mm, a filling rate of 0.12, an average fiber diameter of 1.45 $\mu$m, and a carboxyl group equivalent of 36 $\mu$eq/g.

The obtained fiber material was subjected to polymer coating treatment in the same manner as in Example 1. The carboxyl group equivalent of the nonwoven fabric thus polymer-coated was 36 $\mu$eq/g. The mass of the coat layer per g of the nonwoven fabric was 6.2 mg/g (fiber material + coat layer). The surface $\zeta$ potential of the nonwoven fabric thus coated was 43 mV.

A filter was prepared in the same way as in Example 1 using the nonwoven fabric thus polymer-coated and subjected to the blood test.

As a result, the leukocyte residual rate was $8.7 \times 10^{-3}$, and the filtration time was 10.0 minutes, demonstrating high leukocyte removal performance and a short filtration time. Also, the bradykinin production rate was 1.5, and the C3a activation rate was 1.3, demonstrating that the post-filtration blood has good blood quality.

[Example 9]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m², a thickness of 0.13 mm, a filling rate of 0.12, an average fiber diameter of 1.4 $\mu$m, and a carboxyl group equivalent of 44 $\mu$eq/g.

The obtained fiber material was subjected to polymer coating treatment in the same manner as in Example 1. The carboxyl group equivalent of the nonwoven fabric thus polymer-coated was 44 $\mu$eq/g. The mass of the coat layer per g of the nonwoven fabric was 7.0 mg/g (fiber material + coat layer). The surface $\zeta$ potential of the nonwoven fabric thus coated was 42 mV.

A filter was prepared in the same way as in Example 1 using the nonwoven fabric thus polymer-coated and subjected to the blood test.

As a result, the leukocyte residual rate was $7.9 \times 10^{-3}$, and the filtration time was 11.2 minutes, demonstrating high leukocyte removal performance and a short filtration time. Also, the bradykinin production rate was 1.6, and the C3a activation rate was 1.4, demonstrating that the post-filtration blood has good blood quality.

[Example 10]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.35 $\mu$m, and a carboxyl group equivalent of 52 $\mu$eq/g.

The obtained fiber material was subjected to polymer coating treatment in the same manner as in Example 1. The carboxyl group equivalent of the nonwoven fabric thus polymer-coated was 52 $\mu$eq/g. The mass of the coat layer per g of the nonwoven fabric was 7.8 mg/g (fiber material + coat layer). The surface $\zeta$ potential of the nonwoven fabric thus coated was 44 mV.

A filter was prepared in the same way as in Example 1 using the nonwoven fabric thus polymer-coated and subjected to the blood test.

As a result, the leukocyte residual rate was $7.3 \times 10^{-3}$, and the filtration time was 12.6 minutes, demonstrating high leukocyte removal performance and a short filtration time. Also, the bradykinin production rate was 1.7, and the C3a activation rate was 1.5, demonstrating that the post-filtration blood has good blood quality.

[Example 11]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.3 $\mu$m, and a carboxyl group equivalent of 62 $\mu$eq/g.

The obtained fiber material was subjected to polymer coating treatment in the same manner as in Example 1. The carboxyl group equivalent of the nonwoven fabric thus polymer-coated was 62 $\mu$eq/g. The mass of the coat layer per g of the nonwoven fabric was 8.0 mg/g (fiber material + coat layer). The surface $\zeta$ potential of the nonwoven fabric thus coated was 41 mV.

A filter was prepared in the same way as in Example 1 using the nonwoven fabric thus polymer-coated and subjected to the blood test.

As a result, the leukocyte residual rate was $5.0 \times 10^{-3}$, and the filtration time was 13.5 minutes, demonstrating high leukocyte removal performance and a short filtration time. Also, the bradykinin production rate was 1.8, and the C3a activation rate was 1.6, demonstrating that the post-filtration blood has good blood quality.

[Example 12]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.25 $\mu$m, and a carboxyl group equivalent of 82 $\mu$eq/g.

The obtained fiber material was subjected to polymer coating treatment in the same manner as in Example 1. The carboxyl group equivalent of the nonwoven fabric thus polymer-coated was 82 $\mu$eq/g. The mass of the coat layer per g of the nonwoven fabric was 8.3 mg/g (fiber material + coat layer). The surface $\zeta$ potential of the nonwoven fabric thus coated was 45 mV.

A filter was prepared in the same way as in Example 1 using the nonwoven fabric thus polymer-coated and subjected to the blood test.

As a result, the leukocyte residual rate was $4.7 \times 10^{-3}$, and the filtration time was 14.2 minutes, demonstrating high leukocyte removal performance and a short filtration time. Also, the bradykinin production rate was 1.9, and the C3a activation rate was 1.7, demonstrating that the post-filtration blood has good blood quality.

[Example 13]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.2 $\mu$m, and a carboxyl group equivalent of 92 $\mu$eq/g.

The obtained fiber material was subjected to polymer coating treatment in the same manner as in Example 1. The carboxyl group equivalent of the nonwoven fabric thus polymer-coated was 92 $\mu$eq/g. The mass of the coat layer per g of the nonwoven fabric was 10.3 mg/g (fiber material + coat layer). The surface $\zeta$ potential of the nonwoven fabric thus coated was 42 mV.

A filter was prepared in the same way as in Example 1 using the nonwoven fabric thus polymer-coated and subjected to the blood test.

As a result, the leukocyte residual rate was $3.9 \times 10^{-3}$, and the filtration time was 16.1 minutes, demonstrating high leukocyte removal performance and a short filtration time. Also, the bradykinin production rate was 2.0, and the C3a activation rate was 1.8, demonstrating that the post-filtration blood has good blood quality.

[Example 14]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.1 $\mu$m, and a carboxyl group equivalent of 100 $\mu$eq/g.

The obtained fiber material was subjected to polymer coating treatment in the same manner as in Example 1. The carboxyl group equivalent of the nonwoven fabric thus polymer-coated was 100 $\mu$eq/g. The mass of the coat layer per g of the nonwoven fabric was 12.4 mg/g (fiber material + coat layer). The surface $\zeta$ potential of the nonwoven fabric thus coated was 44 mV.

A filter was prepared in the same way as in Example 1 using the nonwoven fabric thus polymer-coated and subjected to the blood test.

As a result, the leukocyte residual rate was $3.7 \times 10^{-3}$, and the filtration time was 17.2 minutes, demonstrating high leukocyte removal performance and a short filtration time. Also, the bradykinin production rate was 2.2, and the C3a activation rate was 1.9, demonstrating that the post-filtration blood has good blood quality.

[Example 15]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.05 $\mu$m, and a carboxyl group equivalent of 109 $\mu$eq/g.

The obtained fiber material was subjected to polymer coating treatment in the same manner as in Example 1. The carboxyl group equivalent of the nonwoven fabric thus polymer-coated was 109 $\mu$eq/g. The mass of the coat layer per g of the nonwoven fabric was 13.3 mg/g (fiber material + coat layer). The surface $\zeta$ potential of the nonwoven fabric thus coated was 43 mV.

A filter was prepared in the same way as in Example 1 using the nonwoven fabric thus polymer-coated and subjected to the blood test.

As a result, the leukocyte residual rate was $3.5 \times 10^{-3}$, and the filtration time was 18.0 minutes, demonstrating high leukocyte removal performance and a short filtration time. Also, the bradykinin production rate was 2.5, and the C3a activation rate was 2.0, demonstrating that the post-filtration blood has good blood quality.

[Example 16]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 118 $\mu$eq/g.

The obtained fiber material was subjected to polymer coating treatment in the same manner as in Example 1. The carboxyl group equivalent of the nonwoven fabric thus polymer-coated was 118 $\mu$eq/g. The mass of the coat layer per g of the nonwoven fabric was 15.2 mg/g (fiber material + coat layer). The surface $\zeta$ potential of the nonwoven fabric thus coated was 40 mV.

A filter was prepared in the same way as in Example 1 using the nonwoven fabric thus polymer-coated and subjected to the blood test.

As a result, the leukocyte residual rate was 3.2 × 10$^{-3}$, and the filtration time was 23.0 minutes, demonstrating high leukocyte removal performance and a short filtration time. Also, the bradykinin production rate was 2.8, and the C3a activation rate was 2.0, demonstrating that the post-filtration blood has good blood quality.

[Example 17]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature higher than that of Example 1 in order to set the carboxyl group equivalent to a high value. The physical properties of the fiber material thus heat-treated were a basis weight of 22 g/m$^2$, a thickness of 0.13 mm, a filling rate of 0.12, an average fiber diameter of 0.9 $\mu$m, and a carboxyl group equivalent of 136 $\mu$eq/g.

The obtained fiber material was subjected to polymer coating treatment in the same manner as in Example 1. The carboxyl group equivalent of the nonwoven fabric thus polymer-coated was 136 $\mu$eq/g. The mass of the coat layer per g of the nonwoven fabric was 16.0 mg/g (fiber material + coat layer). The surface $\zeta$ potential of the nonwoven fabric thus coated was 46 mV.

A filter was prepared in the same way as in Example 1 using the nonwoven fabric thus polymer-coated and subjected to the blood test.

As a result, the leukocyte residual rate was 3.0 × 10$^{-3}$, and the filtration time was 29.0 minutes, demonstrating high leukocyte removal performance and a short filtration time. Also, the bradykinin production rate was 3.9, and the C3a activation rate was 2.3, demonstrating that the post-filtration blood has good blood quality.

[Comparative Example 1]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 133 $\mu$eq/g. The fiber material was not subjected to polymer coating treatment. The surface $\zeta$ potential of the nonwoven fabric was -57 mV.

A filter was prepared in the same way as in Example 1 using this nonwoven fabric and subjected to the blood test.

As a result, the leukocyte residual rate was 14.3 × 10$^{-3}$, and the filtration time was 15.3 minutes, demonstrating that leukocyte removal performance is low and is practically unsuitable, despite a low filtration time. Also, the bradykinin production rate was 153.1, and the C3a activation rate was 1.6, demonstrating that the post-filtration blood is also practically unsuitable in terms of blood quality due to the high bradykinin production rate.

[Comparative Example 2]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 120 $\mu$eq/g.

The obtained fiber material was subjected to polymer coating treatment in the same manner as in Example 1. The carboxyl group equivalent of the nonwoven fabric thus polymer-coated was 120 $\mu$eq/g. The mass of the coat layer per g of the nonwoven fabric was 3.5 mg/g (fiber material + coat layer). The surface $\zeta$ potential of the nonwoven fabric thus coated was -11 mV.

A filter was prepared in the same way as in Example 1 using this nonwoven fabric and subjected to the blood test.

As a result, the leukocyte residual rate was 8.1 × 10$^{-3}$, and the filtration time was 9.9 minutes, demonstrating high leukocyte removal performance and a short filtration time. On the other hand, the bradykinin production rate was 112.3, and the C3a activation rate was 1.4, demonstrating that the post-filtration blood is practically unsuitable in terms of blood quality due to the high bradykinin production rate.

[Comparative Example 3]

A fiber material was prepared by the method of spinning a fiber assembly made of PET fibers, followed by the heat treatment of the fiber assembly thus spun in the same way as in Example 4. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 24 $\mu$eq/g. The fiber material was not subjected to polymer coating treatment. The surface $\zeta$ potential of the nonwoven fabric was -44 mV.

A filter was prepared in the same way as in Example 1 using this nonwoven fabric and subjected to the blood test.

As a result, the leukocyte residual rate was 18.2 $\times$ 10$^{-3}$, and the filtration time was 16.2 minutes, demonstrating that leukocyte removal performance is low and is practically unsuitable, despite a low filtration time. Also, the bradykinin production rate was 203.8, and the C3a activation rate was 0.9, demonstrating that the post-filtration blood is also practically unsuitable in terms of blood quality due to the high bradykinin production rate.

[Comparative Example 4]

A fiber material was prepared by the method of spinning a fiber assembly made of PET fibers, followed by the heat treatment of the fiber assembly thus spun in the same way as in Example 4 except that the spinning temperature was changed. Specifically, melt spinning was performed at a temperature lower than that of Example 4 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.7 $\mu$m, and a carboxyl group equivalent of 16 $\mu$eq/g.

The obtained fiber material was subjected to polymer coating treatment in the same manner as in Example 1. The carboxyl group equivalent of the nonwoven fabric thus polymer-coated was 16 $\mu$eq/g. The mass of the coat layer per g of the nonwoven fabric was 1.9 mg/g (fiber material + coat layer). The surface $\zeta$ potential of the nonwoven fabric thus coated was 22 mV.

A filter was prepared in the same way as in Example 1 using this nonwoven fabric and subjected to the blood test.

As a result, the leukocyte residual rate was 22.3 $\times$ 10$^{-3}$, and the filtration time was 5.4 minutes, demonstrating that leukocyte removal performance is low and is practically unsuitable, despite a low filtration time. This is probably because an area capable of adsorbing leukocytes was decreased due to the thick average fiber diameter of the fiber material. On the other hand, the bradykinin production rate was 17.8, and the C3a activation rate was 1.7, demonstrating that the post-filtration blood has good blood quality.

[Comparative Example 5]

A fiber material was prepared by the method of spinning a fiber assembly made of PET fibers, followed by the heat treatment of the fiber assembly thus spun in the same way as in Comparative Example 4. The physical properties of the fiber material thus heat-treated were a basis weight of 22 g/m$^2$, a thickness of 0.13 mm, a filling rate of 0.12, an average fiber diameter of 1.7 $\mu$m, and a carboxyl group equivalent of 12 $\mu$eq/g.

The obtained fiber material was subjected to polymer coating treatment in the same manner as in Example 1. The carboxyl group equivalent of the nonwoven fabric thus polymer-coated was 12 $\mu$eq/g. The mass of the coat layer per g of the nonwoven fabric was 11.2 mg/g (fiber material + coat layer). The surface $\zeta$ potential of the nonwoven fabric thus coated was 89 mV.

A filter was prepared in the same way as in Example 1 using this nonwoven fabric and subjected to the blood test.

As a result, the leukocyte residual rate was 17.5 $\times$ 10$^{-3}$, and the filtration time was 6.9 minutes, demonstrating that leukocyte removal performance is low and is practically unsuitable, despite a low filtration time. This is probably because an area capable of adsorbing leukocytes was decreased due to the thick average fiber diameter of the fiber material, as in Comparative Example 4. On the other hand, the bradykinin production rate was 4.9, and the C3a activation rate was 8.7, demonstrating that the post-filtration blood has good blood quality.

[Comparative Example 6]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 153 $\mu$eq/g.

The obtained fiber material was subjected to polymer coating treatment in the same manner as in Example 1. The carboxyl group equivalent of the nonwoven fabric thus polymer-coated was 153 $\mu$eq/g. The mass of the coat layer per g of the nonwoven fabric was 41.6 mg/g (fiber material + coat layer). The surface $\zeta$ potential of the nonwoven fabric thus coated was 38 mV.

A filter was prepared in the same way as in Example 1 using this nonwoven fabric and subjected to the blood test.

As a result, the leukocyte residual rate was $11.3 \times 10^{-3}$, and the filtration time was 18.0 minutes, demonstrating that leukocyte removal performance is low and is practically unsuitable, despite a low filtration time. On the other hand, the bradykinin production rate was 3.5, and the C3a activation rate was 2.2, demonstrating that the post-filtration blood has good blood quality.

[Comparative Example 7]

A fiber material was prepared by the method of spinning a fiber assembly made of PBT fibers, followed by the heat treatment of the fiber assembly thus spun in the same way as in Example 1. The physical properties of the fiber material thus heat-treated were a basis weight of 22 g/m$^2$, a thickness of 0.13 mm, a filling rate of 0.12, an average fiber diameter of 1.0 $\mu$m, and a carboxyl group equivalent of 149 $\mu$eq/g.

The obtained fiber material was subjected to polymer coating treatment in the same manner as in Example 1. The carboxyl group equivalent of the nonwoven fabric thus polymer-coated was 149 $\mu$eq/g. The mass of the coat layer per g of the nonwoven fabric was 62.3 mg/g (fiber material + coat layer). The surface $\zeta$ potential of the nonwoven fabric thus coated was 92 mV.

A filter was prepared in the same way as in Example 1 using this nonwoven fabric and subjected to the blood test.

As a result, the leukocyte residual rate was $4.4 \times 10^{-3}$, and the filtration time was 39.2 minutes, demonstrating that the filtration time is long and is practically unsuitable, despite high leukocyte removal performance. On the other hand, the bradykinin production rate was 79.2, and the C3a activation rate was 14.5, demonstrating that the post-filtration blood is also practically unsuitable in terms of blood quality due to the high C3a activation rate.

[Comparative Example 8]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature lower than that of Example 1 in order to keep the carboxyl group equivalent low. The physical properties of the fiber material thus heat-treated were a basis weight of 22 g/m$^2$, a thickness of 0.13 mm, a filling rate of 0.12, an average fiber diameter of 1.6 $\mu$m, and a carboxyl group equivalent of 16 $\mu$eq/g.

The obtained fiber material was subjected to polymer coating treatment in the same manner as in Example 1. The carboxyl group equivalent of the nonwoven fabric thus polymer-coated was 16 $\mu$eq/g. The mass of the coat layer per g of the nonwoven fabric was 4.1 mg/g (fiber material + coat layer). The surface $\zeta$ potential of the nonwoven fabric thus coated was 46 mV.

A filter was prepared in the same way as in Example 1 using this nonwoven fabric and subjected to the blood test.

As a result, the leukocyte residual rate was $11.2 \times 10^{-3}$, and the filtration time was 7.1 minutes, demonstrating that leukocyte removal performance is low and is practically unsuitable, despite a low filtration time. On the other hand, the bradykinin production rate was 1.2, and the C3a activation rate was 1.1, demonstrating that the post-filtration blood has good blood quality.

[Comparative Example 9]

A fiber material was prepared by the method of spinning a fiber assembly made of PBT fibers, followed by the heat treatment of the fiber assembly thus spun in the same way as in Example 1 except that the spinning temperature was changed. Specifically, melt spinning was performed at a temperature higher than that of Example 1 in order to set the carboxyl group equivalent to a high value. The physical properties of the fiber material thus heat-treated were a basis weight of 22 g/m$^2$, a thickness of 0.13 mm, a filling rate of 0.12, an average fiber diameter of 0.8 $\mu$m, and a carboxyl group equivalent of 145 $\mu$eq/g.

The obtained fiber material was subjected to polymer coating treatment in the same manner as in Example 1. The carboxyl group equivalent of the nonwoven fabric thus polymer-coated was 145 $\mu$eq/g. The mass of the coat layer per g of the nonwoven fabric was 17.1 mg/g (fiber material + coat layer). The surface $\zeta$ potential of the nonwoven fabric thus coated was 41 mV.

A filter was prepared in the same way as in Example 1 using this nonwoven fabric and subjected to the blood test.

As a result, the leukocyte residual rate was $1.9 \times 10^{-3}$, and the filtration time was 32.4 minutes, demonstrating that the filtration time is long and is practically unsuitable, despite high leukocyte removal performance. On the other hand, the bradykinin production rate was 5.1, and the C3a activation rate was 3.4, demonstrating that the post-filtration blood has good blood quality.

The blood evaluation results of Examples 1 to 17 and Comparative Examples 1 to 9 are summarized in Tables 1 to 3.

[Table 1]

| | Number | Example 1 | Example 2 | Example 3 | Example 4 | Example 5 | Example 6 | Example 7 | Example 8 | Example 9 |
|---|---|---|---|---|---|---|---|---|---|---|
| Physical property | Nonwoven fabric base material | PBT | PBT | PBT | PET | PET | PET | PBT | PBT | PBT |
| | Carboxyl group equivalent ($\mu$eq/g) | 122 | 131 | 127 | 26 | 21 | 28 | 27 | 36 | 44 |
| | Average fiber diameter ($\mu$m) | 1.0 | 1.0 | 1.0 | 1.0 | 1.0 | 1.0 | 1.5 | 1.45 | 1.4 |
| | $\zeta$ potential (mV) | 36 | 96 | 123 | 23 | 99 | 132 | 42 | 43 | 42 |
| | Amount of coating (mg/g) | 9.0 | 26.2 | 38.7 | 4.2 | 13.8 | 29.7 | 5.3 | 6.2 | 7.0 |
| Effect | Leukocyte residual rate ($\times$ 10$^{-3}$) | 6.2 | 0.7 | 0.2 | 7.6 | 1.2 | 0.4 | 9.8 | 8.7 | 7.9 |
| | Filtration time (min) | 9.0 | 21.0 | 29.6 | 7.4 | 20.2 | 28.7 | 8.5 | 10.0 | 11.2 |
| | Bradykinin production rate (-) | 3.1 | 1.7 | 1.2 | 11.4 | 3.7 | 2.1 | 1.3 | 1.5 | 1.6 |
| | C3a activation rate (-) | 1.8 | 5.3 | 9.6 | 1.5 | 4.4 | 8.7 | 1.2 | 1.3 | 1.4 |

[Table 2]

| | Number | Example 10 | Example 11 | Example 12 | Example 13 | Example 14 | Example 15 | Example 16 | Example 17 |
|---|---|---|---|---|---|---|---|---|---|
| Physical property | Nonwoven fabric base material | PBT | PBT | PBT | PBT | PBT | PBT | PBT | PBT |
| | Carboxyl group equivalent (μeq/g) | 52 | 62 | 82 | 92 | 100 | 109 | 118 | 136 |
| | Average fiber diameter (μm) | 1.35 | 1.3 | 1.25 | 1.2 | 1.1 | 1.05 | 1.0 | 0.9 |
| | $\zeta$ potential (mV) | 44 | 41 | 45 | 42 | 44 | 43 | 40 | 46 |
| | Amount of coating (mg/g) | 7.8 | 8.0 | 8.3 | 10.3 | 12.4 | 13.3 | 15.2 | 16.0 |
| Effect | Leukocyte residual rate ($\times 10^{-3}$) | 7.3 | 5.0 | 4.7 | 3.9 | 3.7 | 3.5 | 3.2 | 3.0 |
| | Filtration time (min) | 12.6 | 13.5 | 14.2 | 16.1 | 17.2 | 18.0 | 23.0 | 29.0 |
| | Bradykinin production rate (-) | 1.7 | 1.8 | 1.9 | 2.0 | 2.2 | 2.5 | 2.8 | 3.9 |
| | C3a activation rate (-) | 1.5 | 1.6 | 1.7 | 1.8 | 1.9 | 2.0 | 2.0 | 2.3 |

[Table 3]

| | Number | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|---|
| Physical property | Nonwoven fabric base material | PBT | PBT | PET | PET | PET | PBT | PBT | PBT | PBT |
| | Average fiber diameter ($\mu$m) | 1.0 | 1.0 | 1.0 | 1.7 | 1.7 | 1.0 | 1.0 | 1.6 | 0.8 |
| | Carboxyl group equivalent ($\mu$eq/g) | 133 | 120 | 24 | 16 | 12 | 153 | 149 | 16 | 145 |
| | $\zeta$ potential (mV) | -57 | -11 | -44 | 22 | 89 | 38 | 92 | 46 | 41 |
| | Amount of coating (mg/g) | - | 3.5 | - | 1.9 | 11.2 | 41.6 | 62.3 | 4.1 | 17.1 |
| Effect | Leukocyte residual rate ($\times 10^{-3}$) | 14.3 | 8.1 | 18.2 | 22.3 | 17.5 | 11.3 | 4.4 | 11.2 | 1.9 |
| | Filtration time (min) | 15.3 | 9.9 | 16.2 | 5.4 | 6.9 | 18.0 | 39.2 | 7.1 | 32.4 |
| | Bradykinin production rate (-) | 153.1 | 112.3 | 203.8 | 17.8 | 4.9 | 3.5 | 79.2 | 1.2 | 5.1 |
| | C3a activation rate (-) | 1.6 | 1.4 | 0.9 | 1.7 | 8.7 | 2.2 | 14.5 | 1.1 | 3.4 |

As shown in Tables 1 to 3, it was able to be confirmed from the results of Examples 1 to 17 that favorable leukocyte removal performance and filtration time, and good blood quality of post-filtration blood can be achieved by using a polyester fiber material, adjusting the carboxyl group equivalent of a nonwoven fabric to a predetermined range, and positively controlling a surface $\zeta$ potential by coating treatment. By contrast, when the surface $\zeta$ potential of the nonwoven fabric was negative (Comparative Examples 1 to 3), the bradykinin production rate was exceedingly high.

On the other hand, in the case of exceedingly lowering the carboxyl group equivalent of the nonwoven fabric by suppressing the quantity of heat, etc. applied to a resin during fiber material spinning in order to render the surface $\zeta$ potential of the nonwoven fabric positive (Comparative Examples 4, 5, and 8), this resulted in the thick average fiber diameter of the nonwoven fabric and reduced leukocyte removal performance. When the carboxyl group equivalent of the nonwoven fabric was too high (Comparative Examples 6, 7, and 9) and when the amount of coating was exceedingly large for coating in order to positively control the $\zeta$ potential (Comparative Examples 6 and 7), the balance between the leukocyte removal performance and the filtration time was poor. This is probably because the increased amount of coating did not permit uniform coating of the fiber material with the coat layer and hindered uniform permeation of blood into the nonwoven fabric. In Comparative Example 9, clogging, etc. occurred due to too small an average fiber diameter, probably extending a filtration time, though the amount of coating was not exceedingly increased.

Particularly, in the case of performing coating treatment such that the $\zeta$ potential was 0 mV or larger but was not exceedingly high while controlling the carboxyl group equivalent to a high value within the range from 20 to 140 ($\mu$eq/g) (Examples 1 and 12 to 17), preferred results were obtained about all of leukocyte removal performance, a filtration time, suppression of bradykinin production, and suppression of C3a activation, and performance balance was favorable. The high carboxyl group equivalent was able to increase the amount of coating without exceedingly elevating the $\zeta$ potential. This was able to sufficiently hydrophilize the nonwoven fabric, probably improving leukocyte removal performance without casing the activation of C3a. Furthermore, it is considered that the elevated coating rate of the coat layer consequently suppressed the drift phenomenon of blood and also kept the filtration time at the predetermined value or lower.

In all of Examples in which the $\zeta$ potential was 0 mV or larger, the effect of suppressing the production of bradykinin was able to be confirmed. When the $\zeta$ potential was controlled at 100 mV or lower, the filtration time was further shortened, and the C3a activation rate was also able to be suppressed. It was therefore able to be confirmed that the $\zeta$ potential adjusted to a proper range (from 0 to 100 mV) is practically more desirable (see Examples 2, 3, 5, and 6).

When PET and PBT are compared as a material constituting the fiber material of the nonwoven fabric, it was suggested that equivalent performance can be achieved by controlling the carboxyl group equivalent and the $\zeta$ potential of the nonwoven fabric within the preferred ranges. On the other hand, PBT tends to have a higher carboxyl group equivalent than that of PET even if the fiber materials have similar physical properties (basis weight, thickness, filling rate, average fiber diameter, etc.).

Industrial Applicability

The filter element of the present invention can be used for removing unnecessary components (e.g., aggregates, pathogenic substances (viruses, bacteria, protozoa, infected red cells, etc.), and drugs for blood processing) contained in blood, in addition to leukocytes.

Particularly, the filter element of the present invention can further suppress the production of bradykinin and the activation of undesirable components such as complements as compared with conventional filters, while maintaining equivalent performance in terms of basic performance such as removal performance for leukocytes and the like and a filtration time as compared with the conventional filters. Thus, the filter element of the present invention is considered to have great industrial applicability because the filter element of the present invention can achieve excellent blood quality without influencing actual use conditions as compared with conventional filter elements.

Reference Signs List

1 ... Container, 3 ... First port (liquid inlet/outlet), 4 ... Second port (liquid inlet/outlet), 5 ... Filter element, 7 ... Space on the first port side, 8 ... Space on the second port side, 9 ... Outer edge of the filter element, 10 ... Blood processing filter.

**Claims**

1. A filter element (5) for a blood processing filter (10), comprising a nonwoven fabric, **characterized in that** the nonwoven fabric has a carboxyl group equivalent of from 20 to 140 $\mu$eq/g and a surface $\zeta$ potential of 0 mV or larger, wherein the surface $\zeta$ potential of the nonwoven fabric is measured by the following procedure:

(i) the nonwoven fabric is cut into a size of approximately 1 cm × 2 cm and dipped in a 1 mM potassium chloride solution,

(ii) the nonwoven fabric thus dipped is loaded in a cell for plate samples, followed by electrophoresis using a $\zeta$ potential apparatus, in this context, the migration solution used is a dispersion of polystyrene latex particles in a 1 mM potassium chloride solution,

(iii) the sample thus electrophoresed is analyzed using software attached to the apparatus from the mobility of the latex particles shown in the spectrum to calculate the surface $\zeta$ potential (mV) of the nonwoven fabric.

2. The filter element (5) for a blood processing filter (10) according to claim 1, wherein the carboxyl group equivalent of the nonwoven fabric is from 54 to 140 $\mu$eq/g.

3. The filter element (5) for a blood processing filter (10) according to claim 1 or 2, wherein the filter element (5) comprises the nonwoven fabric having a basic nitrogen-containing functional group in a surface portion.

4. The filter element (5) for a blood processing filter (10) according to claim 3, wherein
the surface portion further has a nonionic group, and
a ratio of an amount of substance of the basic nitrogen-containing functional group to a total amount of substance of the nonionic group and the basic nitrogen-containing functional group is from 0.2 to 50.0% by mol.

5. The filter element (5) for a blood processing filter (10) according to any of claims 1 to 4, wherein the nonwoven fabric comprises a fiber material, and the carboxyl group equivalent of the fiber material is from 20 to 140 $\mu$eq/g.

6. The filter element (5) for a blood processing filter (10) according to any of claims 1 to 5, wherein the fiber material is made of a polyester resin.

7. The filter element (5) for a blood processing filter (10) according to any of claims 1 to 6, wherein the nonwoven fabric comprises a coat layer.

8. A blood processing filter (10) comprising a filter element (5) according to any of claims 1 to 7.

9. A leukocyte removal method comprising a step of allowing a leukocyte-containing liquid to pass through a blood processing filter (10) according to claim 8, under the proviso that methods for the treatment of the human and animal body by surgery or therapy are excluded.

## Patentansprüche

1. Filterelement (5) für einen Blutverarbeitungsfilter (10), der einen Vliesstoff umfasst, **dadurch gekennzeichnet, dass** der Vliesstoff weist ein Carboxygruppenäquivalent von 20 bis 140 $\mu$äq/g und ein Oberflächenpotential $\zeta$ von 0 mV oder mehr auf, wobei das Oberflächenpotential $\zeta$ des Vliesstoffes durch das folgende Verfahren gemessen wird:

(i) der Vliesstoff wird auf eine Größe von ungefähr 1 cm x 2 cm geschnitten und in eine 1 mM Kaliumchloridlösung eingetaucht;
(ii) der so eingetauchte Vliesstoff wird in eine Zelle für Plattenproben geladen, und dann erfolgt eine Elektrophorese unter Verwendung einer $\zeta$-Potential-Vorrichtung, und in diesem Zusammenhang ist die verwendete Migrationslösung eine Dispersion von Polystyrol-Latexteilchen in einer 1 mM Kaliumchloridlösung;
(iii) die so elektrophorierte Probe wird mit Hilfe von an die Vorrichtung gebundener Software analysiert, um aus der im Spektrum gezeigten Mobilität der Latexteilchen das Oberflächenpotential $\zeta$ (mV) des Vliesstoffes zu berechnen.

2. Filterelement (5) für einen Blutverarbeitungsfilter (10) gemäß Anspruch 1, wobei das Carboxygruppenäquivalent des Vliesstoffes 54 bis 140 $\mu$äq/g beträgt.

3. Filterelement (5) für einen Blutverarbeitungsfilter (10) gemäß Anspruch 1 oder 2, wobei das Filterelement (5) den Vliesstoff umfasst, der in einem Oberflächenteil eine basischen Stickstoff enthaltende funktionelle Gruppe aufweist.

4. Filterelement (5) für einen Blutverarbeitungsfilter (10) gemäß Anspruch 3, wobei der Oberflächenteil weiterhin eine nichtionische Gruppe aufweist und das Verhältnis der Stoffmenge der basischen Stickstoff enthaltenden funktionellen Gruppe zur gesamten Stoffmenge der nichtionischen Gruppe und der basischen Stickstoff enthaltenden funktionellen Gruppe 0,2 bis 50,0 Mol-% beträgt.

**5.** Filterelement (5) für einen Blutverarbeitungsfilter (10) gemäß einem der Ansprüche 1 bis 4, wobei der Vliesstoff ein Fasermaterial umfasst und das Carboxygruppenäquivalent des Fasermaterials 20 bis 140 μäq/g beträgt.

**6.** Filterelement (5) für einen Blutverarbeitungsfilter (10) gemäß einem der Ansprüche 1 bis 5, wobei das Fasermaterial aus einem Polyesterharz besteht.

**7.** Filterelement (5) für einen Blutverarbeitungsfilter (10) gemäß einem der Ansprüche 1 bis 6, wobei der Vliesstoff eine Hüllschicht umfasst.

**8.** Blutverarbeitungsfilter (10), der ein Filterelement (5) gemäß einem der Ansprüche 1 bis 7 umfasst.

**9.** Leukocytenentfernungsverfahren, umfassend einen Schritt des Passierens einer leukocytenhaltigen Flüssigkeit durch einen Blutverarbeitungsfilter (10) gemäß Anspruch 8, mit der Maßgabe, dass Verfahren zur Behandlung des menschlichen und tierischen Körpers durch Chirurgie oder Therapie ausgeschlossen sind.

**Revendications**

**1.** Élément de filtre (5) pour un filtre de traitement du sang (10), comprenant une étoffe non tissée, **caractérisé en ce que** l'étoffe non tissée a un équivalent de groupes carboxyle de 20 à 140 μeq/g et un potentiel $\zeta$ de surface de 0 mV ou plus, dans lequel le potentiel $\zeta$ de surface de l'étoffe non tissée est mesuré par la procédure suivante:

    (i) l'étoffe non tissée est découpée en une taille d'environ 1 cm $\times$ 2 cm et trempée dans une solution de chlorure de potassium 1 mM,
    (ii) l'étoffe non tissée ainsi trempée est chargée dans une cellule pour échantillons en plaques, ce qui est suivi par une électrophorèse à l'aide d'un appareil à potentiel $\zeta$, dans ce contexte, la solution de migration utilisée est une dispersion de particules de latex de polystyrène dans une solution de chlorure de potassium 1 mM,
    (iii) l'échantillon ainsi soumis à une électrophorèse est analysé à l'aide d'un logiciel joint à l'appareil d'après la mobilité des particules de latex montrée dans le spectre pour calculer le potentiel $\zeta$ de surface (mV) de l'étoffe non tissée.

**2.** Élément de filtre (5) pour un filtre de traitement du sang (10) selon la revendication 1, dans lequel l'équivalent de groupes carboxyle de l'étoffe non tissée est de 54 à 140 μeq/g.

**3.** Élément de filtre (5) pour un filtre de traitement du sang (10) selon la revendication 1 ou 2, dans lequel l'élément de filtre (5) comprend l'étoffe non tissée ayant un groupe fonctionnel contenant de l'azote basique dans une partie de surface.

**4.** Élément de filtre (5) pour un filtre de traitement du sang (10) selon la revendication 3, dans lequel
la partie de surface a en outre un groupe non ionique, et
un rapport d'une quantité de substance du groupe fonctionnel contenant de l'azote basique à une quantité de substance totale du groupe non ionique et du groupe fonctionnel contenant de l'azote basique est de 0,2 à 50,0% en moles.

**5.** Élément de filtre (5) pour un filtre de traitement du sang (10) selon l'une quelconque des revendications 1 à 4, dans lequel l'étoffe non tissée comprend un matériau fibreux, et l'équivalent de groupes carboxyle du matériau fibreux est de 20 à 140 μeq/g.

**6.** Élément de filtre (5) pour un filtre de traitement du sang (10) selon l'une quelconque des revendications 1 à 5, dans lequel le matériau fibreux est fait d'une résine de polyester.

**7.** Élément de filtre (5) pour un filtre de traitement du sang (10) selon l'une quelconque des revendications 1 à 6, dans lequel l'étoffe non tissée comprend une couche de revêtement.

**8.** Filtre de traitement du sang (10) comprenant un élément de filtre (5) selon l'une quelconque des revendications 1 à 7.

9. Procédé de retrait des leucocytes comprenant une étape de permettre à un liquide contenant des leucocytes de passer à travers un filtre de traitement du sang (10) selon la revendication 8, à condition que les procédés pour le traitement du corps humain et animal par chirurgie ou thérapie soient exclus.

Fig. 1

Fig. 2

**EP 3 501 561 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015088019 A
- JP 4261623 B
- JP 2007054212 A
- JP 4134043 B

**Non-patent literature cited in the description**

- **KAGAKU BINRAN.** Handbook of Chemistry in English), Basics II. Maruzen Publishing Co, 1975, 164
- *Japanese Journal of Clinical Medicine,* 1995, vol. 53